(19) Europäisches Patentamt
European Patent Office
Office européen des brevets

(11) **EP 3 699 574 A1**

(12) **EUROPEAN PATENT APPLICATION**

(43) Date of publication:
**26.08.2020 Bulletin 2020/35**

(51) Int Cl.:
**G01N 15/14** *(2006.01)* **G01N 35/00** *(2006.01)*
**G01N 15/10** *(2006.01)* **G01N 1/40** *(2006.01)*

(21) Application number: **20157372.2**

(22) Date of filing: **14.02.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.02.2019 JP 2019028833**

(71) Applicant: **SYSMEX CORPORATION
Kobe-shi
Hyogo 651-0073 (JP)**

(72) Inventors:
• **Takahashi, Yusuke**
  **Hyogo, 651-0073 (JP)**
• **Yanagida, Masatoshi**
  **Hyogo, 651-0073 (JP)**
• **Shirai, Kentaro**
  **Hyogo, 651-0073 (JP)**
• **Ijiri, Yuichi**
  **Hyogo, 651-0073 (JP)**
• **Iwanaga, Shigeki**
  **Hyogo, 651-0073 (JP)**

(74) Representative: **Hoffmann Eitle
Patent- und Rechtsanwälte PartmbB
Arabellastraße 30
81925 München (DE)**

(54) **MEASUREMENT SUCCESS/FAILURE DETERMINATION METHOD AND SAMPLE MEASUREMENT DEVICE**

(57)     The present invention provides a method for determining a success or failure of a measurement of a target particle contained in a measurement sample. The method includes: detecting the target particle and other particle other than the target particle in the measurement sample; and determining the success or failure of the measurement of the target particle based on at least a detection result of the other particle.

Operation of sample measurement device

FIG. 7

EP 3 699 574 A1

**Description**

FIELD OF THE INVENTION

The present invention relates to a measurement success/failure determination method and a sample measurement device.

BACKGROUND

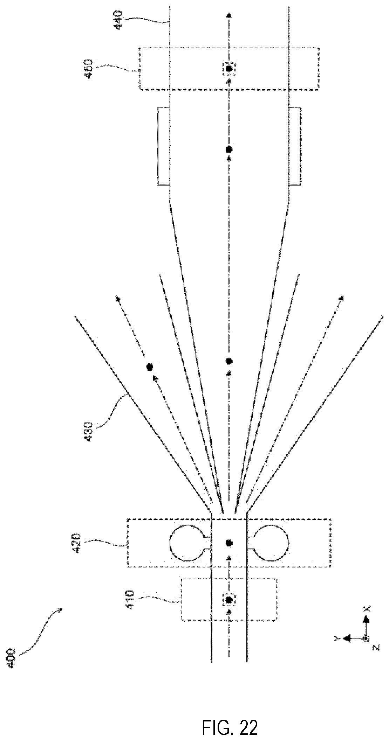

In recent years, measurement of particles contained in a sample has been performed for various purposes. For example, as cells contained in a blood sample reflect various states of a living body, measurement of cells contained in a sample collected from a subject is performed for grasping a status of a disease of the subject. Japanese Patent Application Publication No. 2017-116558 describes a particle imaging device 400 for imaging a circulating tumor cell (CTC) contained in peripheral blood (see FIG. 22). CTC is a tumor cell released into the blood from a primary tumor or the like, and it is known that a small number of tumor cells circulate in the body of a cancer patient along with a blood flow.

In the particle imaging device 400 described in Japanese Patent Application Publication No. 2017-116558, particles having a low possibility of CTC among particles included in the sample based on the detection result of the particles in the particle detection unit 410 are caused to flow through a flow path 430 provided outside by the particle selection unit 420. Particles having a high possibility of CTC based on the detection result of the particles by the particle detection unit 410 are caused to flow through the centrally provided flow path 440, and images of the particles are captured by a particle imaging unit 450.

SUMMARY OF THE INVENTION

Here, when desiring to accurately measure the number of target particles contained in a sample and various problems occur during the detection of the particles such that the particles cannot be detected for a fixed period, the number of target particles contained in the sample cannot be accurately estimated from the detection results. For example, when scarce particles in a sample are detected as target particles as in the case of CTC, it is important to measure the entire amount of the sample so that the target particles can be detected completely. However, even if the detection fails and some particles are missed, it cannot be determined from the detection result whether the number of detected target particles is small due to the fact that the number of target particles contained in the sample is actually small, or the number of detected target particles is small due to the fact that the total amount of the sample is not measured because of the detection failure. For this reason, in order to properly evaluate the state of the subject based on the detection result of the particles, it is necessary to first grasp whether the detection of the particles has been correctly performed.

Therefore, the present invention provides a measurement success/failure determination method and a sample measurement device capable of determining whether a measurement of a sample has been correctly performed even when scarce particles included in the sample are targeted.

A first aspect of the present invention relates to a measurement success/failure determination method for determining the success or failure of measurement in measuring target particles contained in a measurement sample. The measurement success/failure determination method according to this aspect detects target particles and particles other than the target particles in the measurement sample, and determines the success or failure of the measurement of the target particles based on at least a result of detecting the other particles.

The inventors have found that when rare particles included in a measurement sample are measured as target particles, the detection results of particles other than the target particles reflect the success or failure of the measurement of the target particles. Therefore, according to the measurement success/failure determination method according to this aspect, the success or failure of the measurement of the target particle can be determined based on at least the detection result of other particles.

When the success or failure of the measurement can be determined in this way, it is possible to determine whether the number of detected target particles is small because the actual number of target particles contained in the measurement sample is small, or the number of detected target particles is small due to a detection defect. Therefore, the target particles contained in the measurement sample can be appropriately evaluated based on the success or failure of the measurement. In this way, for example, in the determination of a disease state, it is possible to reduce false negatives caused by the small number of target particles due to a measurement failure.

Although a detection failure may occur each time the measurement is performed, the success or failure of the measurement is determined based on the detection result in the actually performed measurement according to the measurement success/failure determination method of the present aspect. Therefore, it can be determined whether the measurement has been correctly performed for each measurement of the measurement sample.

In the measurement success/failure determination method according to this aspect, in the determination of the success of the measurement, the success or failure of the measurement of the target particle also may be determined based on the detection result of the target particles and other particles detected in the particle detection. The measurement sample includes not only the target particles, but also particles that are not the target particles. Therefore, the success or failure of the measurement of the target particles may be determined based on the detection results of both the target particles and the other particles.

The measurement success/failure determination method according to this aspect calculates a value related to a detection rate of the target particles detected in the measurement based on at least a result of detection of other particles, and determines whether the measurement of the target particles is successful based on the value related to the detection rate. The value relating to the detection rate of the target particles may be a numerical value that reflects the percentage of the target particles contained in the measurement sample the measurement sample is measured, for example, the detection rate of all particles contained in the measurement sample, or the detection rate of only particles other than the target particles.

In this case, the measurement success/failure determination method according to this aspect includes the calculation of a value related to the detection rate, wherein at least an estimated value related to the number of other particles expected to be detected in the measurement is calculated based on at least the number of other particles detected per unit time, and a value related to the detection rate may be calculated based on at least the estimated value and the number of other particles actually detected in the measurement. The estimated value in this case may include at least the number of other particles expected to be detected in the measurement, and may include the number of target particles expected to be detected in the measurement.

In this case, the measurement success/failure determination method according to this aspect includes calculating a value related to the detection rate, wherein a value related to the detection rate also may be calculated based on the ratio of the estimated value and the number of at least other particles actually detected in the measurement.

Alternatively, the measurement success/failure determination method according to this aspect includes calculating a value related to the detection rate wherein the value related to the detection rate is calculated based on the ratio of the number of at least other particles actually detected in the measurement for the estimated value.

The measurement success/failure determination method according to the present aspect includes calculating a value related to the detection rate, wherein an approximation expression that approximates a temporal change in the number of at least the other particles detected in the measurement is calculated based on at least the number of the other particles detected per unit time, and an estimated value related to at least the number of other particles expected to be detected in the measurement is calculated based on the approximation expression.

In this case, the measurement success/failure determination method according to this aspect also may calculate the approximation expression by excluding a time zone in which at least other detected particles are equal to or less than a predetermined number in calculating the approximation expression.

In the measurement success/failure determination method according to this aspect, an approximation expression may be calculated by excluding a time zone before a predetermined timing from all the time zones in which the measurement sample is measured. In this way a detection failure can be determined even when a failure occurs unintentionally immediately after the start of measurement.

In the measurement success/failure determination method according to this aspect, an approximation expression may be calculated by excluding a time zone after a predetermined timing from all the time zones in which the measurement sample is measured. In this way it is possible to determine a detection failure even when the particles settle unintentionally.

In the measurement success/failure determination method according to this aspect, in the determination of the success or failure of the measurement, a value related to the detection rate may be compared with a predetermined threshold value to determine the success or failure of the measurement of the target particles.

In this case, the measurement success/failure determination method according to this aspect also may change the predetermined threshold value in accordance with input by the user. In this way the threshold value can be set in accordance with the request of the hospital, the practice of the testing facility, and the like.

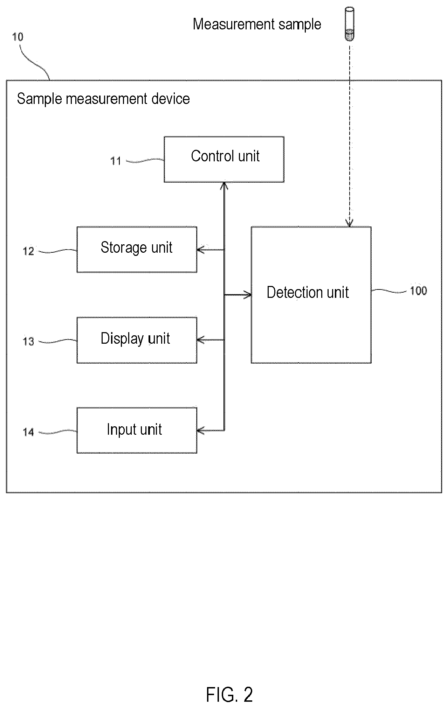

Referring to FIG. 2, in the measurement success/failure determination method according to the present aspect, the measurement success or failure determination result also may be displayed on a display unit (13). In this way the operator can smoothly comprehend the success or failure of the measurement by referring to the determination result displayed on the display unit.

In this case, the measurement success/failure determination method according to the present aspect also may display a graph showing the change over time in the number of at least the other particles actually detected in the measurement on the display unit (13). In this way the operator can comprehend the measurement status by referring to the graph displayed on the display unit.

The measurement success/failure determination method according to this aspect, wherein, in calculating the value related to the detection rate, an estimated value related to the value of the detection signal expected to be detected in the measurement is calculated based on the value of the detection signal obtained from at least the other particles detected per unit time, and a value related to the detection rate is calculated based on the estimated value and the value of a detection signal actually detected in the measurement.

In the measurement success/failure determination method according to this aspect, in the calculation of the value related to the detection rate, a value related to the detection rate also may be calculated based on the time during which the number of other particles detected per unit time is at least a predetermined number and/or information of the time when the number of other particles detected per unit time is at least a predetermined number.

In the measurement success/failure determination method according to this aspect, in the detection of target particles and other particles, a measurement sample is caused to flow in a flow path (111), and the target particles and other particles in the measurement sample flowing in the flow path (111) are detected.

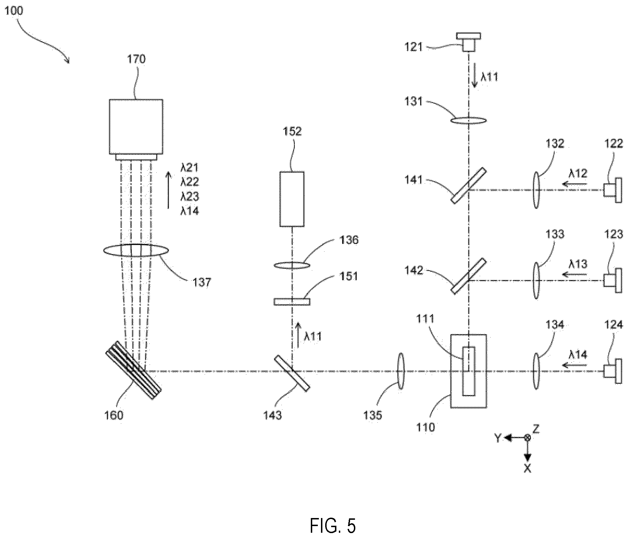

Referring to FIG. 5, in the measurement success/failure determination method according to this aspect, in the detection of target particles and other particles, a measurement sample is caused to flow through a flow path (111) formed in a flow cell (110), and the measurement sample flowing through the sample is irradiated with light, whereupon the light given off by the measurement sample is detected.

The measurement success/failure determination method according to this aspect includes detecting target particles and other particles in the detection of target particles and other particles contained in the measurement sample based on a plurality of images, calculating an estimated value related to the at least the number of other particles expected to be detected in the measurement based on at least the number of other particles detected in each image in the calculation of the value related to the detection rate, and calculating a value related to a detection rate based on the estimated value, and at least the number of other particles actually detected in the measurement. In this way, for example, when detection is performed using a fluorescence microscope, the success or failure of the measurement can be determined by the measurement success/failure determination method.

In the measurement success/failure determination method according to this aspect, the target particles and the other particles are, for example, cells.

In the measurement success/failure determination method according to this aspect, the number of target particles in the measurement sample may be smaller than the number of other particles in the measurement sample.

In the measurement success/failure determination method according to this aspect, the target particles are, for example, circulating tumor cells in blood, and the other particles are leukocytes.

In the measurement success/failure determination method according to this aspect, the measurement sample is prepared by excluding some leukocytes contained in the blood based on the difference in the size of the circulating tumor cells and leukocytes in the blood, and then determining the success or failure of the measurement of the target particles based on the detection result of the leukocytes that were not removed in the preparation of the measurement sample when determining the success or failure of the measurement.

Referring to FIG. 2, in the measurement success/failure determination method according to this aspect, a value related to the number of target particles also may be displayed on the display unit (13). In this way, a physician or the like can make a diagnosis of the patient by referring to the value related to the number of target particles.

A second aspect of the present invention relates to a sample measurement device for measuring target particles contained in a measurement sample. The sample measurement device (10) according to this aspect includes a detection unit (100) that detects target particles and particles other than the target particles in the measurement sample, and at least a target particle based on a detection result of the other particles, and a control unit (11) that determines whether the measurement of the target particle is successful based on at least the detection result of the other particles.

The detection unit, for example, includes an optical detection unit that irradiates light on a measurement sample flowing through a flow path provided in a flow cell and images or measures the light generated from the measurement sample, and an electric resistance type detection unit that applies current to the measurement sample flowing through the flow path and measures the particles based on changes in electric resistance.

According to the sample measurement device of the present aspect, the same effect as in the first aspect is achieved.

A third aspect of the present invention relates to a measurement success/failure determination method. The measurement success/failure determination method according to this aspect detects particles in the measurement sample, calculates an estimated value regarding the number of particles expected to be detected in the measurement based on the number of particles detected per unit time, calculates a value related to the detection rate based on the estimated value and the number of particles actually detected in the measurement, and determines the success or failure of the particle measurement based on the value related to the detection rate.

According to the measurement success/failure determination method according to this aspect, the detection rate is a numerical value reflecting the ratio of particles contained in the measurement sample that can be detected in the measurement of the measurement sample.

Therefore, according to the measurement success/failure determination method of this aspect, the success or failure of the particle measurement can be determined based on the value related to the detection rate.

According to the present invention, it is possible to determine whether the measurement of the sample has been properly performed in the measurement of rare target particles contained in a sample.

BRIEF DESCRIPTION OF THE DRAWINGS

FIG. 1 is a flowchart showing an outline of a measurement success/failure determination method according to Embodiments 1 to 4;

FIG. 2 is a block diagram showing a structure of a sample measurement device according to the first embodiment;

FIG. 3 is a flowchart showing the sequence of pretreatment according to the first embodiment;

FIG. 4A is a schematic diagram showing the structure of a chip according to the first embodiment; FIG. 4B is a schematic diagram showing a cross section of the micro flow path according to the first embodiment; FIG. 4C is a schematic diagram showing an end part on the output end side of the micro flow path according to the first embodiment;

FIG. 5 is a schematic diagram showing the structure of a detection unit according to the first embodiment;

FIG. 6 is a schematic diagram showing the structure of a detection unit for flowing a measurement sample, reference fluid, and sheath liquid to the flow cell according to the first embodiment;

FIG. 7 is a flowchart showing the operation of the sample measurement device according to the first embodiment;

FIGS. 8A to 8C are graphs showing the number of measured particles for explaining acquisition of an estimated value and acquisition of a detection rate according to the first embodiment;

FIG. 9 is a flowchart illustrating details of a process of acquiring an estimated value according to the first embodiment;

FIG. 10 is a flowchart illustrating details of a detection rate acquisition step according to the first embodiment;

FIGS. 11A to 11C are graphs of the number of measured particles acquired in the second verification of the measurement success/failure determination method according to the first embodiment;

FIGS. 12A to 12C are graphs of the number of measured particles obtained in the second verification of the measurement success/failure determination method according to the first embodiment

FIG. 13 is a flowchart showing details of the measurement success/failure determination process according to the first embodiment.

FIG. 14 is a schematic diagram showing the structure of a screen displayed on a display unit in a display process according to the first embodiment;

FIG. 15 is a schematic diagram showing the structure of a screen displayed on a display unit in a display process according to the first embodiment;

FIG. 16 is a scattergram created in the first verification of the measurement success/failure determination method according to the first embodiment;

FIG. 17 is a detection rate distribution diagram created in the second verification of the measurement success/failure determination method according to the first embodiment;

FIGS. 18A and 18B are graphs of the number of measured particles acquired in the second verification of the measurement success/failure determination method according to the first embodiment;

FIGS. 19A to 19C are graphs showing the number of measured particles for explaining acquisition of an estimated value and acquisition of a detection rate according to a second embodiment;

FIGS. 20A to 20C are graphs showing the number of measured particles for explaining acquisition of an approximation expression according to a third embodiment;

FIGS. 21A and 21B are graphs showing the number of measured particles for explaining acquisition of a detection rate according to a fourth embodiment; and

FIG. 22 is a schematic diagram for explaining a configuration according to the related art.

DESCRIPTION OF THE PREFERRED EMBODIMENTS

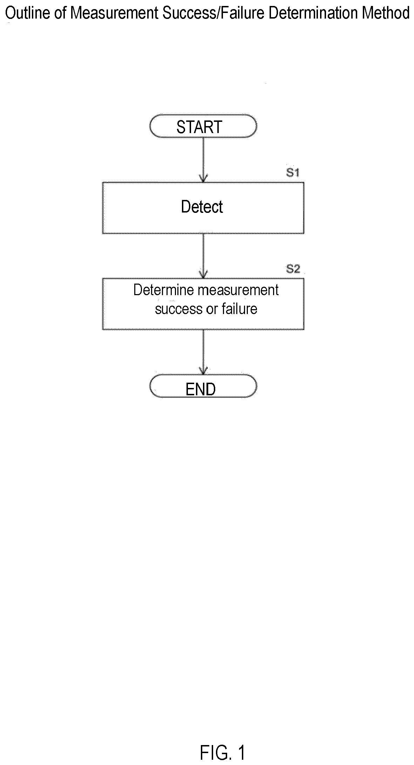

An outline of the measurement success/failure determination method of the present invention will be described with reference to FIG. 1. Below, an example will be described in which CTC target particles contained in a measurement sample prepared from peripheral blood is measured. In this measurement, the leukocytes contained in the measurement sample are regarded as particles other than the target particles, and the success or failure of the measurement of the target particles is determined based at least on the detection result of the leukocytes.

In detection step S1, target particles and particles other than the target particles in the measurement sample are detected. In this way an image of the particle is captured, or the data of the detection signal based on the light or the like generated from the particle is obtained.

Subsequently, in measurement success/failure determination step S2, the success or failure of the measurement of the target particles is determined based on the data acquired in the detection step S1. In determination step S2 for determining the success or failure of the measurement, for example, the following steps are performed.

First, an estimated value that is expected to be obtained is calculated from the data of the detection signal obtained in the detection step S1 and, for example, a value related to the detection rate of the target particles is calculated based on the ratio of an actual measurement value such as the number of particles actually detected in the measurement or the value of the detection signal and the estimated value that is originally expected to be obtained. Details of the calculation of the value related to the detection rate of the target particles will be described later. Then, the success or failure of the measurement of the target particles is determined by comparing the value related to the detection rate of the target particles with a predetermined threshold value. For example, when the value related to the detection rate of the target particles is the ratio of the actually measured value to the estimated value, and the value related to the detection rate of the target particles is larger than the threshold value, it is determined that the particles in the measurement sample have been sufficiently detected and the measurement of the target particles is determined to have been appropriate.

Here, the measurement sample prepared from blood or the like includes not only target particles to be analyzed such as CTC but also particles other than target particles such as leukocytes. For example, even when the target particles are rare cells and the number of target particles contained in the measurement sample is small, the number of particles contained in the measurement sample becomes a certain quantity by including other particles. In this way the success or failure of target particle measurement can be properly determined using the value related to the detection rate of the target particles since the value related to the detection rate of the target particles increases or decreases according to the success or failure of the measurement regardless of the number of the target particles depending on whether the measurement is appropriate or not appropriate.

Note that although the measurement success/failure determination method is assumed to be performed using a sample measurement device, a part or all of each step may be performed by an operator via a technique.

First Embodiment

Structure of Sample Measurement Device 10

FIG. 2 is a block diagram showing the structure of the sample measurement device 10. The sample measurement device 10 includes a control unit 11, a storage unit 12, a display unit 13, an input unit 14, and a detection unit 100.

The control unit 11 is configured by a CPU. The control unit 11 performs various processes based on a program stored in the storage unit 12. The control unit 11 is connected to each unit in the sample measurement device 10, receives signals from each unit, and controls the operation of each unit. The storage unit 12 includes a RAM, a ROM, a hard disk, and the like. The display unit 13 includes a liquid crystal display, a plasma display, a CRT (Cathode Ray Tube) display, and the like. The input unit 14 includes a mouse, a keyboard, and the like. Note that the display unit 13 and the input unit 14 may be integrally configured by a touch panel display. The detection unit 100 is an optical unit for detecting particles in the measurement sample prepared by the pretreatment. Details of the detection unit 100 will be described later.

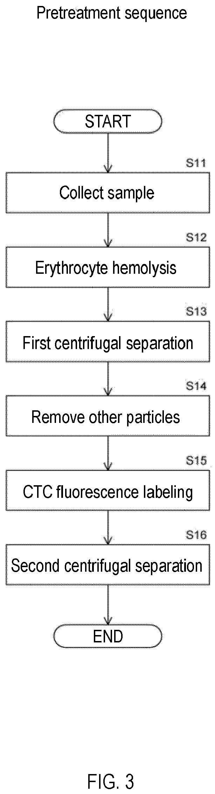

FIG. 3 is a flowchart showing the sequence of pretreatment for preparing a measurement sample to be provided to the sample measurement device 10.

Note that although the case where the sample is a whole blood sample and the target particles are CTC in the whole blood sample is described below, the pretreatment of the measurement sample is appropriately set depending on the type of the sample, the target particles, and the like, and the present invention is not limited to the following method. The sample collected from the subject is not limited to whole blood, and may be any sample containing target particles corresponding to the analysis, such as urine and bone marrow fluid. The target particle is a rare cell derived from the subject, and is not limited to CTC, and may be a cell such as a vascular endothelial cell (CEC), a hematopoietic stem cell (HSC), a fetal erythroblast, and particles such as exosomes and microparticles.

In the sample collection step S11, for example, 5 mL of whole blood is collected from the subject as a sample. Whole blood collected from a subject includes erythrocytes, platelets, leukocytes, and CTC. In the erythrocyte hemolysis step S12, erythrocytes are lysed with respect to whole blood collected from the subject using a predetermined reagent containing a surfactant or the like. Subsequently, in a first centrifugation step S13, the sample in which the erythrocytes have been lysed is centrifuged by a centrifuge, and the supernatant is removed. In this way a sample with almost no erythrocytes is obtained.

Subsequently, in the other particle removal step S14, the sample from which erythrocytes have been substantially removed is caused to flow through the spiral flow path, so that CTC as the target particle and other non-CTC particles are separated based on the difference in particle size. That is, other particles such as leukocytes and platelets are removed from the sample obtained in the first centrifugation step S13, and a sample having an increased CTC concentration is obtained.

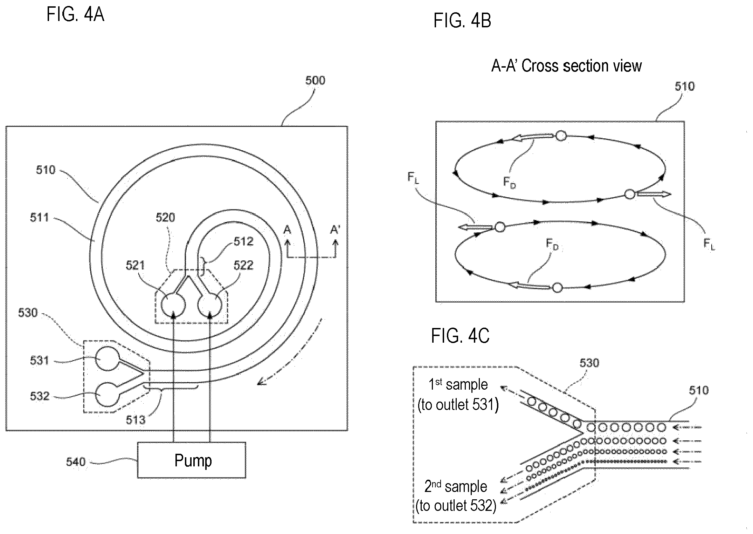

When obtaining a sample having an increased CTC concentration in the other non-target particle removing step S14, for example, the chip 500 shown in FIG. 4A is used.

FIG. 4A is a schematic diagram showing the structure of the chip 500.

The sample obtained in the first centrifugation step S13 is caused to flow through the micro flow path 510 provided in the chip 500, and the chip 500 separates the sample obtained in the first centrifugation step S13 into a first sample and a second sample.

The chip 500 includes a micro flow path 510, an input end 520, and an output end 530. A micro flow path is generally a flow path having a depth and width in the range of 10 $\mu$m to 1000 $\mu$m. The chip 500 is configured by, for example, overlaying another plate-like member on a thin plate-like member in which grooves corresponding to the micro flow path 510, the input end 520, and the output end 530 are formed. The other non-target particle removing step S14 is based on a separation principle called Dean Flow Fractionation.

The micro flow path 510 has a spiral curved part 511 and two straight parts 512 and 513. The shape of the curved part 511 is an arc when viewed in a direction perpendicular to the chip 500. The shape of the two straight parts 512 and 513 is straight when viewed in a direction perpendicular to the chip 500. The input end 520 is provided at the end of the micro flow path 510 on the center side of the spiral shape, and the output end 530 is provided at the end of the micro flow path 510 opposite to the input end 520. The input end 520 is connected to the curved part 511 via the straight part 512, and the output end 530 is connected to the curved part 511 via the straight part 513.

The input end 520 has two inlets 521 and 522. The two inlets 521 and 522 are configured by holes provided in a member configuring the chip 500. The inlet 521 is connected to the curved part 511 via the straight part 512 on the spiral outer peripheral side of the micro flow path 510. The inlet 522 is connected to the curved part 511 via the straight part 512 on the inner peripheral side of the spiral shape of the micro flow path 510. The output end 530 has two outlets 531 and 532. The two outlets 531 and 532 are configured by holes provided in a member configuring the chip 500. The outlet 531 is connected to the curved part 511 via the straight part 513 on the inner circumferential side of the spiral shape of the micro flow path 510. The outlet 532 is connected to the curved part 511 via the straight part 513 on the outer peripheral side of the spiral shape of the micro flow path 510.

The sample obtained in the first centrifugation step S13 is injected into the inlet 521, and a sheath liquid for flowing the sample is injected into the inlet 522. The amount of the sample injected into the inlet 521 is, for example, 4 mL, and the amount of the sheath liquid injected into the inlet 522 is, for example, 45 mL. Then, a positive pressure is applied to the two inlets 521 and 522 by a pump 540 connected to the two inlets 521 and 522 via a tube. In this way the sample injected into the inlet 521 and the sheath liquid injected into the inlet 522 flow in the micro flow channel 510 toward the output end 530. The amount of the first sample obtained from the outlet 531 is, for example, 10 mL, and the amount of the second sample obtained from the outlet 532 is, for example, 39 mL.

FIG. 4B is a cross-sectional view taken along the line A-A' of the micro flow path 510 shown in FIG. 4A. In FIG. 4B, the right side is the outer peripheral side of the spiral shape, and the left side is the inner peripheral side of the spiral shape.

When the sample obtained in the first centrifugation step S13 flows through the micro flow path 510, a force is generated within the micro flow path 510 which changes the position of the flowing particles in a direction orthogonal to the direction of the flow path, as shown in FIG. 4B. Specifically, a lift force FL and a Dean drag force FD are generated and have different magnitudes depending on the diameter of the particles are generated. Generally, in a micro flow path, the flow velocity is distributed such that the velocity at the center of the cross section of the path is large and the velocity near the wall is small. In the spiral micro flow path 510, a secondary flow called a Dean vortex is generated due to the above-mentioned flow velocity distribution, as shown by an elliptical arrow in FIG. 4B. Dean drag FD and lift FL can be expressed by the following equations (1) and (2).

Equations

$$F_D = 3\pi\mu \overline{U}_{Dean} a_p \qquad (1)$$

$$F_L = \rho G^2 C_L a_p^4 \qquad (2)$$

$\mu$: Viscosity of fluid   $\rho$ : Fluid density   $\overline{U}_{Dean}$ : Dean speed

$G$ : Shear speed   $a_p$ : Particle size   $C_L$ : Lift coefficient

$$G = U_{max} / D_h$$

$U_{max}$ : Maximum flow velocity of micro channel    $D_h$ :Hydraulic diameter

The particles in the sample are distributed according to the diameter value in the micro flow path 510 by the lift FL and the Dean drag FD while being swept down along the micro flow path 510.

FIG. 4C is a schematic diagram showing an end of the micro flow path 510 on the output end 530 side.

When a Dean vortex is generated in the micro flow path 510, as shown in FIG. 4C, particles are distributed according to the diameter value at the output end 530 side end of the micro flow path 510. In this way a first sample containing particles having a predetermined diameter or more is obtained from the outlet 531, and a second sample containing particles having a diameter smaller than the predetermined diameter is obtained from the outlet 532.

The diameter of most leukocytes is below a value approximately 10-15 μm, and the diameter of most CTCs is above the value approximately 10-15 μm. Therefore, when removing leukocytes, platelets, and the residual erythrocytes that were not lysed as other non-CTC particles from the sample obtained in the first centrifugation step S13, the shape of the micro flow path in the chip 500 is set particles having a diameter less than a cutoff value around 10 μm to 15 μm, and retrieving particles having a diameter equal to or greater than the cutoff value.

Here, the amount of CTC circulating in the blood is extremely small, and it is generally said that even a cancer patient has only a few to about a thousand in 10 mL of blood. Therefore, the amount of CTC contained in the first sample is also extremely small. Although the second sample containing particles other non-CTC is removed using the above-mentioned chip 500, some residual leukocytes still remain since there are many leukocytes having the same size as CTC.

In the above-described example, removal of erythrocytes is not limited to using a hemolytic agent, since CTC and erythrocytes also may be separated by the spiral shaped flow path based on the difference in diameter between CTC and erythrocytes.

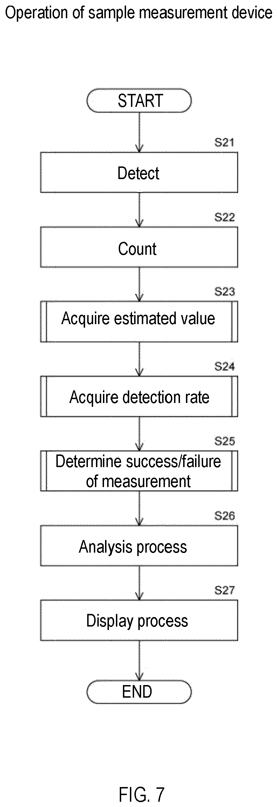

Returning to FIG. 3, in the CTC fluorescent labeling step S15, the CTC target site is fluorescently labeled in the first sample obtained in the non-CTC particle removing step S14. For example, target sites for fluorescent labeling are the nucleus, the Her2 gene, and the centromere region of chromosome 17 (CEP17). Note that the target site to be fluorescently labeled also may be a cell site. The target site is not limited to a gene or a nucleus and may be, for example, a protein of a cell, a cytoplasm, a cell membrane, or a surface antigen on a cell membrane. The fluorescent labeling of the target site is not limited to be performed based on in situ hybridization or specific staining, and may be performed by immunostaining based on an antigen-antibody reaction. When the target site is a gene, it is not limited to the Her2 gene or CEP17, and may be another gene region. When the target site is fluorescently labeled in this way, target particles contained in the measurement sample can be identified and analyzed in analysis step S26 in the sample measurement device 10 described later with reference to FIG. 7.

Subsequently, in a second centrifugation step S16, a process such as centrifugation is performed on the sample containing CTC in which the target site is fluorescently labeled, and the supernatant after centrifugation is removed. In this way the amount of liquid decreases while CTC contained in the sample remains, such that the concentration of CTC increases. A measurement sample to be provided to the sample measurement device 10 is thus obtained.

As described above, the erythrocytes are substantially removed by the erythrocyte hemolysis step S12 and the first centrifugation step S13, and the platelets and the unhemolyzed erythrocytes remaining are substantially removed by the other particle removal step S14. On the other hand, the leukocytes will remain in the sample even after the other particle removal step S14. Specifically, the measurement sample contains several to several hundred CTCs, whereas the measurement sample contains about 10,000 to 200,000 leukocytes. As described above, the measurement sample obtained by the pretreatment contains many leukocytes as other particles.

Here, in the subsequent analysis, CTC is a target particle to be analyzed, whereas leukocytes are another particle that is not to be analyzed. Therefore, leukocytes are essentially unnecessary particles from an analytical point of view. However, in measurement success/failure determination step S25 described with reference to FIG. 7, whether the measurement of the target particles by the sample measurement device 10 has been properly performed is determined using the leukocyte count value that was originally unnecessary for analysis. The determination of the success or failure of the measurement will be described later with reference to FIG. 7.

FIG. 5 is a schematic diagram showing the structure of the detection unit 100.

The detection unit 100 includes a flow cell 110, four light sources 121 to 124, seven condenser lenses 131 to 137, three dichroic mirrors 141 to 143, an optical grating 151, a photodetector 152, a reflection unit 160, and an imaging unit 170. FIG. 5 shows XYZ axes orthogonal to each other.

The measurement sample flows in the flow path 111 of the flow cell 110 in the positive Z-axis direction. The four light sources 121 irradiate light on the measurement sample flowing through the flow cell 110. The four light sources 121 to 124 are configured by semiconductor laser light sources. Light emitted from the four light sources 121 to 124 is laser light having wavelengths λ11, λ12, λ13, and λ14 which are mutually different. The four condenser lenses 131 to 134 collect light emitted from the four light sources 121 to 124, respectively. The dichroic mirror 141 transmits light having the wavelength λ11 and reflects light having the wavelength λ12. The dichroic mirror 142 transmits light of two wavelengths λ11 and λ12 and reflects light having a wavelength λ13. In this way the light of the three wavelengths λ11, λ12, and λ13 is irradiated on the measurement sample flowing through the flow path 111 in the positive direction of the X-axis. The light having the wavelength λ14 is applied to the measurement sample flowing through the flow path 111 in the positive Y-axis direction.

Here, the fluorescent stain that labels the nucleus and the two genes, respectively, is selected from among a fluorescent stain that generates fluorescence of wavelength λ21 when irradiated with excitation light of wavelength λ11, a fluorescent stain that emits light of wavelength λ22 when irradiated with excitation light of wavelength λ12, and fluorescent stain that generates fluorescence at a wavelength λ23 when irradiated with excitation light having a wavelength λ13. By capturing the fluorescence of each of the three wavelengths λ21, λ22, and λ23, fluorescence images of the nucleus and two genes as the target sites can be obtained.

When the measurement sample flowing through the flow cell 110 is irradiated with light of three wavelengths λ11, λ12, and λ13, fluorescence is generated from the fluorescent stains that label the target sites of CTC. When the measurement sample flowing through the flow cell 110 is irradiated with light having the wavelength λ14, the light passes through the cells. The light of wavelength λ14 transmitted through the cells is used to generate a bright-field image. The condenser lens 135 collects light of four wavelengths λ21, λ22, λ23, and λ14 generated from the measurement sample.

Here, a reference liquid containing reference particles flows along with the measurement sample into the flow cell 110. The reference particles are different from the particles contained in the measurement sample, and are non-biological particles used for monitoring the flow velocity in the flow path 111. The reference particles are, for example, a polymer such as a latex or an inorganic substance. The reference particles have an optical property that, when irradiated with light, generate scattered light stronger than other particles such as cells contained in the measurement sample.

When the light of wavelength λ11 is irradiated on the reference particles flowing through the flow path 111 together with the measurement sample, scattered light is generated from the reference particles. The dichroic mirror 143 reflects scattered light of wavelength λ11 generated from the reference particles and transmits light in a wavelength band other than wavelength λ11. The optical grating 151 has a grating structure in which transparent portions and opaque portions are alternately arranged in the Z-axis direction. When the scattered light having the wavelength λ11 enters the optical grating 151, the intensity of the scattered light is modulated by the optical grating 151. The condenser lens 136 collects the modulated light generated by the optical grating 151. A photodetector 152 receives the modulated light condensed by the condenser lens 136. The photodetector 152 is composed of, for example, a photomultiplier tube or a photodiode. The control unit 11 (see FIG. 2) calculates the flow rate of the measurement sample flowing in the flow cell 110 in the Z-axis direction based on the detection signal of the photodetector 152.

The reflection unit 160 has a configuration in which four dichroic mirrors are combined. The four dichroic mirrors of the reflection unit 160 reflect the light of the four wavelengths λ21, λ22, λ23, and λ14 at slightly different angles from each other, and separate the light on the light receiving surface of the imaging unit 170. The condenser lens 137 collects the light reflected by the reflection unit 160.

The imaging unit 170 is configured by a TDI (Time Delay Integration) camera. The imaging unit 170 images fluorescence of three wavelengths λ21, λ22, and λ23, and light of wavelength λ14 based on the flow velocity obtained from the reference particles, and generates a bright-field image corresponding to the light of wavelength λ14, and fluorescence images corresponding to the three wavelengths λ21, λ22, and λ23 as particles of the measurement sample. The control unit 11 stores the fluorescence images and the bright-field image generated by the imaging unit 170 in the storage unit 12.

Here, the imaging unit 170 is configured by a TDI camera. The light received by the light receiving surface of the imaging unit 170 is integrated based on the flow velocity calculated using the photodetector 152 to generate fluorescence images and a bright field image. Thereby, the quality of the fluorescence image and the bright-field image can be improved. When the captured image is acquired by the imaging unit 170, the particles contained in the measurement sample can be accurately analyzed using the captured image in the subsequent analysis step S26 (see FIG. 7).

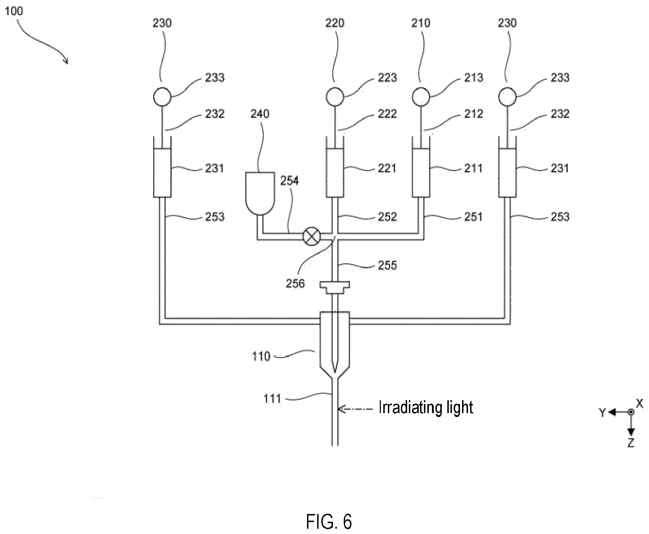

FIG. 6 is a schematic diagram illustrating a configuration of the detection unit 100 for flowing the measurement sample, the reference liquid, and the sheath liquid into the flow cell 110.

The detecting unit 100 includes a first liquid sending unit 210, a second liquid sending unit 220, a third liquid sending unit 230, a chamber 240, and five flow paths 251 to 255.

The first liquid sending unit 210 is configured to send a measurement sample to the flow cell 110. The first liquid sending unit 210 includes a syringe 211, an actuator 212, and a driving mechanism 213. The actuator 212 is inserted into the syringe 211, and includes a plunger, a piston, and the like. The drive mechanism 213 moves the actuator 212 and is configured by a motor or the like.

The second liquid sending unit 220 sends the reference liquid containing the reference particles to the flow cell 110. The second liquid sending unit 220 is also configured similarly to the first liquid sending unit 210, and includes a syringe 221, an actuator 222, and a driving mechanism 223. Two third liquid sending units 230 are provided for the flow cell 110, and send the sheath liquid to the flow cell 110. The third liquid sending unit 230 is configured similarly to the first liquid sending unit 210, and includes a syringe 231, an actuator 232, and a drive mechanism 233. When the sheath liquid is sent, both of the third liquid sending units 230 send the sheath liquid alternately, and while one of the third liquid sending units 230 sends the sheath liquid, the other third liquid sending unit 230 fills the syringe 231 with the sheath liquid. In this way the sheath liquid is sent to the flow cell 110 without interruption.

The first liquid sending unit 210, the second liquid sending unit 220, and the third liquid sending unit 230 may be a diaphragm pump, and also may send the liquid using air pressure produced by an electropneumatic converter connected to a pressure source such as a compressor.

The measurement sample prepared in the pretreatment step is accommodated in the chamber 240. The flow path 251 connects the first liquid sending unit 210 and the connection point 256. The flow path 252 connects the second liquid sending unit 220 and the connection point 256. The flow path 253 connects the third liquid sending unit 230 and the flow cell 110. The flow path 254 connects the chamber 240 and the connection point 256. The flow path 255 connects the connection point 256 and the flow cell 110.

At the time of detection by the detection unit 100, the first liquid sending unit 210 transfers the measurement sample in the chamber 240 through the two flow paths 251 and 254, to a medial position between the connection point 256 and the first liquid sending unit 210. Subsequently, one of the two third liquid sending units 230 sends the sheath liquid to the flow cell 110 via the flow path 253, and the second liquid sending unit 220 sends the reference liquid containing the reference particles to the flow cell 110 through the two flow paths 252 and 255. When the control unit 11 (refer to FIG. 2) determines that the flow rate in the flow path 111 of the flow cell 110 has reached a predetermined value based on the detection signal of the photodetector 152 (refer to FIG. 5), the first liquid sending unit 210 sends the measurement sample drawn into the flow path 251 to the flow cell 110 through the two flow paths 251 and 255. Then, the measurement sample flowing through the flow path 111 of the flow cell 110 is irradiated with light of four wavelengths $\lambda 11$, $\lambda 12$, $\lambda 13$, and $\lambda 14$, as described above. Operation of Sample Measurement Device 10

FIG. 7 is a flowchart showing the operation of the sample measurement device 10 of a first embodiment. Each step shown in FIG. 7 is performed by an operator inputting an instruction to the sample measurement device 10, and executing each step according to the input instruction. Each of the steps shown in FIG. 7 may be automatically performed by the sample measurement device 10, or some or all of the steps shown in FIG. 7 may be manually performed by an operator.

In the detection step S21, the control unit 11 of the sample measurement device 10 shown in FIG. 2 drives the detection unit 100 shown in FIG. 5 to transfer a predetermined amount of the measurement sample prepared in the pretreatment process into the flow path 111 of the flow cell 110, and detect the particles contained in the measurement sample flowing through the path 111. Specifically, the imaging unit 170 shown in FIG. 5 captures a fluorescence image and a bright-field image from each particle included in the measurement sample flowing through the flow path 111 during a prescribed detection period. The control unit 11 shown in FIG. 2 stores the captured fluorescence image and bright-field image in the storage unit 12 together with information related to the time at which the image was captured. Here, the number of leukocytes contained in the measurement sample is about 10,000 to about 200,000, and the number of CTCs contained in the measurement sample is about several to several hundreds, as described above. As described above, most of the particles detected in the detection step S21 are leukocytes since leukocytes are the majority of particles in the measurement sample.

In the measurement step S22, the control unit 11 counts the number of particles for each frame based on the bright-field image stored in the storage unit 12 together with the time-related information, and acquires the number of particles for each frame. One frame corresponds to a unit time, for example, 20 seconds. The number of particles can be accurately measured by using a bright-field image. In the first embodiment, most of the particles detected in the detection step S21 are leukocytes, so the count value obtained in the measurement step S22 is substantially based on leukocytes.

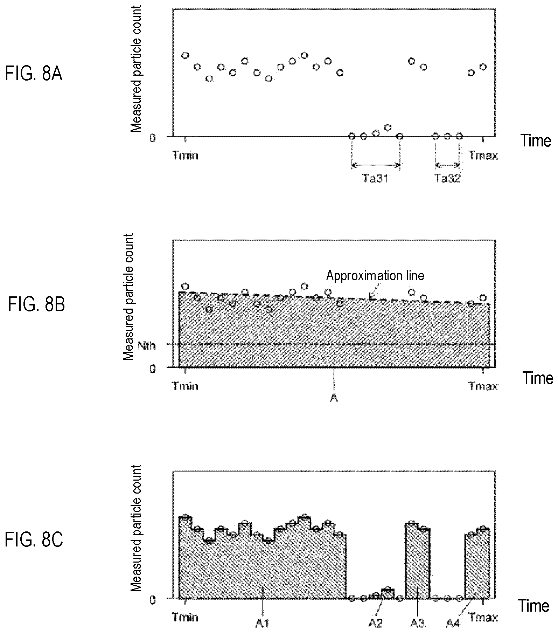

Note that the predetermined amount of the measurement sample refers to a part or all of the measurement sample prepared in the pretreatment. Since CTC, which is a rare cell, is the object of analysis in the first embodiment, it is preferable that the entire amount of the measurement sample is flowed through the flow cell 110 and detected by the detection unit 100. Although the detection unit 100 according to the first embodiment is an optical detection unit that irradiates light on a measurement sample flowing through the flow path 111 provided in the flow cell 110 and measures the light generated from the measurement sample, the present invention is not limited to this configuration inasmuch as an electric resistance type detection unit that supplies an electric current to the measurement sample flowing through the flow path and detects particles based on a change in electric resistance also may be used. FIG. 8A is a graph of the number of measured particles schematically showing the number of particles detected per unit time in the measurement step S22. In each graph, the horizontal axis shows the elapsed time in the measurement time, and the vertical axis shows the number of particles measured in each unit time.

In the measurement step S22, the control unit 11 counts the number of particles per unit time based on the bright-field image acquired by the imaging unit 170. One plot on the graph shows the number of particles measured during a unit time of 20 seconds. As shown in FIG. 8A, the liquid supply of the measurement sample also starts at time Tmin located at the left end of the graph, and ends at time Tmax located at the right end of the graph. In other words, the detection of the entire amount of the measurement sample is completed in the period from Tmin to Tmax. In the example shown in FIG. 8A, although there is a time zone in which the particles are stably detected, particles are not detected in a portion of the time zones Ta31 and Ta32, and there is a possibility that a may occur in detection.

Such a detection failure may occur, for example, when there is a disturbance in the liquid sent to the sheath fluid due to switching both the third liquid supply units 230 for supplying the sheath liquid shown in FIG. 6. A detection failure also may occur when the particles are not identified due to a focus shift of the imaging unit 170 illustrated in FIG. 5, when the integration speed of the TDI camera and flow velocity obtained based on the reference particles cannot be properly synchronized, when light is not properly emitted from the light source 124, when a bright-field image is not properly generated in the imaging unit 170 and the like.

Note that FIGS. 8B and 8C are graphs based on the graph of FIG.8A. The graphs of FIGS. 8B and 8C will be described later in an estimated value acquiring step and a detection rate acquiring step S24.

Returning to FIG. 7, in the estimated value acquiring step S23, the control unit 11 calculates an estimated value related to the number of particles expected to be detected in the detection step S21 based on the number of particles acquired per unit time in the measurement step S22.

In the estimated value acquiring step S23, the control unit 11 calculates and acquires an approximation expression from the measurement data corresponding to the graph of the number of measured particles. In the case of the graph of the number of measured particles shown in FIG. 8A, the control unit 11 excludes a time zone in which there is a possibility that a failure has occurred in detection, and an approximation expression of the number of measured particles is acquired based on the number of measured particles in other time zones, as shown in FIG. 8B. The approximation expression is a straight line that approximates the temporal change in the number of detected particles. Then, the control unit 11 acquires an estimated value based on the approximation expression.

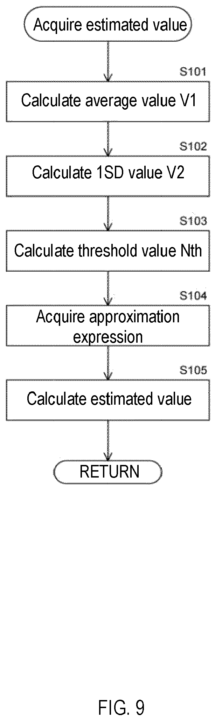

FIG. 9 is a flowchart showing details of the estimated value acquiring step S23.

In the average value calculation step S101, the control unit 11 calculates the average V1 of the number of measured particles in a period in which the number of measured particles in a unit time is 1 or more based on the measurement data corresponding to the graph of FIG. 8Ao. Subsequently, in the 1SD value calculation step S102, the control unit 11 calculates a 1SD value V2 of the number of measured particles in a period in which the number of measured particles per unit time is 1 or more. Subsequently, in the threshold value calculation step S103, the control unit 11 calculates the threshold value Nth by subtracting the value V2 calculated in the 1SD value calculation step S102 from the average V1 calculated in the average value calculation step S101. The thin broken straight line shown in FIG. 8B indicates the threshold value Nth.

Here, the average V1 is a value indicating how many measured particles are acquired per unit time in a time zone in which it is determined that no problem occurs in detection. The value V2 is a value indicating the degree to which the number of measured particles fluctuates with respect to the average V1 in a time period in which it is determined that no problem occurs in the detection. Therefore, the threshold value Nth calculated from the average V1-value V2 indicates the lower limit of 1SD of the range of the value of the number of measured particles in the time zone in which it is determined that no problem occurs in the detection. If the number of measured particles is equal to or greater than the threshold Nth, it is determined that particles have been detected in the target time zone, and if the number of measured particles is less than the threshold Nth, it is determined that particles have not been detected in the target time zone.

Subsequently, in the approximation expression acquiring step S104, the control unit 11 extracts only the number of measured particles equal to or greater than the threshold value Nth from the graph of FIG. 8A as shown in the graph of FIG. 8B, and acquires a linear regression equation of the number of measured particles as an approximate equation based on the data corresponding to the graph shown in FIG. 8B. The thick broken straight line indicates the linear regression equation. As described above, the first-order linear regression equation is obtained excluding the time period in which it is determined that no particles are detected in the measurement. Specifically, this approximation expression represents the number of particles expected to be detected in 20 seconds from T seconds to T + 20 seconds.

Note that although the linear regression expression is used for the calculation of the approximation expression, the present invention is not limited to this configuration, for example, a polynomial approximation also may be performed. The threshold value Nth for excluding a time zone in which there is a possibility that a failure has occurred in detection also is not limited to being calculated by the above-described calculation procedure.

Subsequently, in the estimated value calculating step S105, the control unit 11 acquires, as the estimated value, the total number of particles defined by an approximation expression as shown in FIG. 8B. This estimated value means an estimated value of the number of particles expected to be detected in the present measurement, that is, an estimated value of the total number of particles that would have been obtained without detection failure.

Specifically, the control unit 11 calculates an estimated value by integrating the approximation expression acquired in the approximation expression acquiring step S104 from Tmin to Tmax. Here, one plot on the graph of FIG. 8B shows the number of particles detected in 20 seconds. Therefore, when integrating an approximation expression, it is necessary to reduce the approximation expression to 1/20 in the vertical axis direction in advance. For example, when the time in the horizontal axis direction is T and the number of measured particles in the vertical axis direction is N, the approximate expression is expressed as N = -aT + b. In this case, when the approximation expression is reduced to 1/20 in the vertical axis direction, the newly set approximation expression is N = (- aT + b) / 20. The control unit 11 integrates the approximation expression reduced to 1/20 in this manner from Tmin to Tmax to calculate an estimated value.

The number of measured particles plotted on the graph includes the number of measured target particles and the number of measured other non-target particles. Therefore, the estimated value calculated in the estimated value calculating step S105 is the sum of the number of target particles expected to be detected in the measurement and the number of other particles expected to be detected in the measurement. As described above, the estimated value only needs to include at least the number of other non-target particles expected to be detected in the measurement, and may include the number of target particles expected to be detected in the measurement.

This estimated value corresponds to the area A of a square defined by an approximation expression, a vertical straight line corresponding to the time Tmin, a vertical straight line corresponding to the time Tmax, and a horizontal straight line corresponding to the measured particle number = 0. The area A is the number of particles expected to be detected in the measurement when there is no problem in the detection, and is an estimated value reflecting the total number of particles in the measurement sample. In this way the acquisition of the estimated value in the estimated value acquiring step S23 ends.

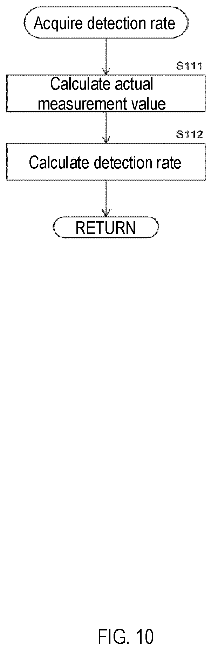

Returning to FIG. 7, in the detection rate acquiring step S24, the control unit 11 determines the detection rate based on the estimated value acquired in the estimation value acquiring step S23 and the number of particles actually detected in the detection step S21. FIG. 10 is a flowchart showing details of the detection rate acquiring step S24.

In the actual measurement value calculation step S111, the control unit 11 calculates the number (actual measurement value) of the particles actually detected in the measurement. The number of particles actually detected in the measurement is the total number of measured particles per unit time. In the example shown in FIG. 8C, the total number of measured particles per unit time corresponds to the area A1+A2+A3+A4.

Subsequently, in the detection ratio calculation step S112, the control unit 11 calculates the detection rate based on the ratio of the total number of particles (estimated value) in the measurement sample and the number of particles actually detected in the measurement (calculated in the actually measured value calculation step S111). The estimated value acquired in the estimated value acquiring step S23 shown in FIG. 7 is used as the total number of particles in the measurement sample. Specifically, the control unit 11 obtains a detection rate by dividing the number of actually detected particles (actually measured value) by the number of measured particles (estimated value) expected when there is no failure. In the examples shown in FIGS. 8A to 8C, the detection rate corresponds to (A1+A2+A3+A4)/A. In this way the detection rate acquisition in the detection rate acquiring step S24 is completed.

Note that in the detection rate calculation step S112 the calculated detection rate is the detection rate of all particles contained in the measurement sample since the estimated value and the actually measured value used for the calculation are values based on both the target particles and the other particles. However, the detection rate is not limited to this configuration, and the detection rate calculated in the detection rate calculation step S112 also may be a value related to the detection rate of the target particles, that is, a numerical value that reflects the proportion of the target particles contained in the measurement sample that can be detected in the measurement of the measurement sample. For example, the detection rate calculated in the detection rate calculation step S112 also may be a detection rate of only other non-target particles.

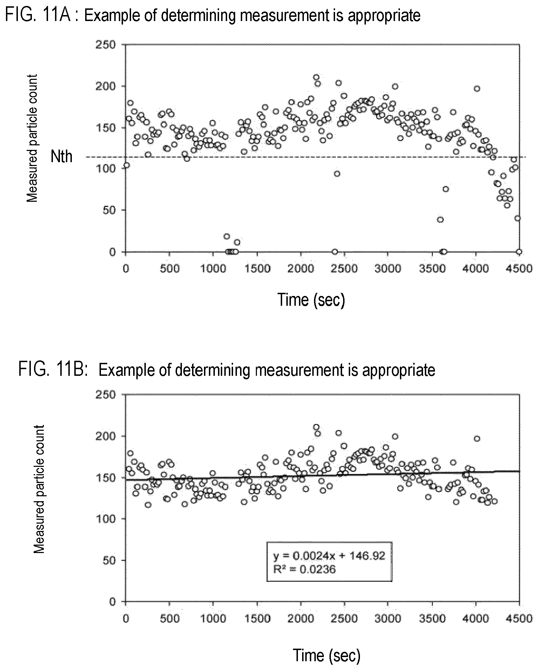

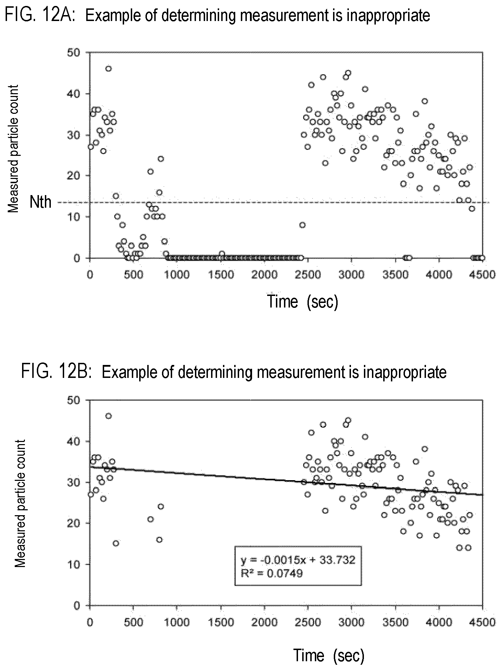

Here, referring to FIGS. 11A and 11B and FIGS. 12A and 12B, we shall describe the sequence of a detection rate obtained based on a graph actually obtained by the inventors in the verification of the measurement success/failure determination method.

FIGS. 11A and 11B and FIGS. 12A and 12B are graphs showing the number of particles detected per unit time in the measurement step S22. In the graph shown in FIG. 11A, around 150 cells are stably measured in most of the time zones located above the straight line of the threshold value Nth indicated by the broken line. In the example shown in FIG. 12A, the time zone located below the straight line of the threshold value Nth indicated by the broken line is longer, and there is a possibility that a failure may occur in the detection as a whole. Note that the sending the liquid of the measurement sample starts at 0 second located at the left end of the graph, and ends at 4500 seconds located at the right end of the graph.

In the case of the graph of the number of measured particles shown in FIG. 11A, the average V1 of the number of measured particles in which the number of measured particles is 1 or more was 144.2. The value V2 of 1SD of the number of measured particles in which the number of measured particles was 1 or more was 30.7. The threshold value Nth was calculated based on the average VI-value V2, and was set to 113.4. The graph of FIG. 11B is obtained by extracting only the number of measured particles equal to or larger than the threshold value Nth from the graph of FIG. 11A. In the graph of FIG. 11B, a primary linear regression equation of the number of measured particles was obtained as indicated by a thick straight line. In this case, the linear regression equation was y = 0.0024x + 146.92, and the coefficient of determination was 0.0236.

Subsequently, the total number of particles defined by the approximation expression shown in FIG. 11B is obtained as an estimated value. The area is calculated based on a square defined by a straight line based on the approximation expression, a vertical straight line corresponding to time = 0, a vertical straight line corresponding to time = 4500, and a horizontal straight line corresponding to the measured particle number = 0. In the graph of FIG. 11B, the area corresponding to the estimated value was 34269.2. In the graph of FIG. 11A, the total number of measured particles in all time zones was 31,140. Therefore, the detection rate was 31140 / 34269.2 = 90.9%.

In the case of the graph of the number of measured particles shown in FIG. 12A, the average V1 of the number of measured particles in which the number of measured particles is 1 or more was 25.1. The value V2 of 1SD of the number of measured particles in which the number of measured particles was 1 or more was 11.3. The threshold value Nth was calculated based on the average VI-value V2, and was set to 13.8. The graph of FIG. 12B is obtained by extracting only the number of measured particles equal to or larger than the threshold value Nth from the graph of FIG. 12A. In the graph of FIG. 12B, a first-order linear regression equation of the number of measured particles was obtained as indicated by a thick straight line. In this case, the first-order linear regression equation was y = -0.0015x + 33.732, and the coefficient of determination was 0.0749.

Subsequently, the total number of particles defined by the approximation expression shown in FIG. 12B is obtained as an estimated value. In the graph of FIG. 12B, the area corresponding to the estimated value was 6827.92. In the graph of FIG. 12A, the total number of measured particles in all time zones was 3409. Therefore, the detection rate was 3409 / 6827.92 = 49.9%.

Returning to FIG. 7, in the determination step S25 of the success or failure of the measurement, the control unit 11 determines the success or failure of the measurement of the target particles by comparing the detection rate with a threshold value Rth for the determination.

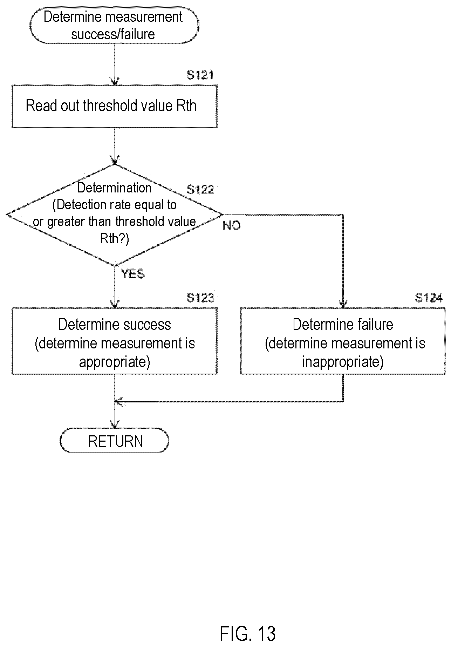

FIG. 13 is a flowchart showing details of the determination step S25 of the success or failure of the measurement.

In the threshold value reading step S121, the control unit 11 reads the threshold value Rth from the storage unit 12 (see FIG. 2). The threshold value Rth is determined based on how many particles must be detected. For example, when the target value of the detection rate is set to 80%, it is considered preferable that the detection rate is equal to or more than -2SD of 80%, that is, 64% or more. Therefore, the threshold value Rth is set to 64%. Note that the threshold value Rth is not limited to the target value of -2SD or more, and may be the target value of -1SD or more. Since the threshold value Rth varies depending on the operation of the facility using the sample measurement device 10, the control unit 11 also may change the threshold value Rth in accordance with an input made by the operator using the input unit 14 (see FIG. 2). In this way the threshold value Rth can be set according to the needs of the hospital, the operation of the testing facility, and the like.

In the determination step S122, the control unit 11 determines whether the detection rate acquired in the detection rate acquiring step S24 shown in FIG. 7 is equal to or greater than the threshold value Rth read out in the threshold value reading step S121. When the detection rate is equal to or larger than the threshold value Rth, in the appropriateness determination step S123, the control unit 11 determines that the measurement of the target particles is correct if the detection performed in the detection step S21 shown in FIG.7 is appropriate. On the other hand, when the detection rate is less than the threshold value Rth in the inappropriateness determination step S124, the control unit 11 determines that the measurement of the target particles was incorrect if the detection performed in the detection step S21 shown in FIG. 7 was inappropriate.

When the threshold value Rth is set to 64%, in the examples shown in FIGS. 11A and 11B, the detection rate is 90.9%, and the control unit 11 determines that the measurement of the target particles is correct. On the other hand, in the examples shown in FIGS. 12A and 12B, the control unit 11 determines that the measurement of the target particles is inappropriate since the detection rate is 49.9%.

When the success or failure of the measurement of the target particles is determined in this way, it is possible to determine whether the number of target particles detected was small because the number of target particles contained in the measurement sample was actually small, or whether the number of detected target particles was small due to detection failure. Therefore, if the success or failure of the measurement of the target particles is obtained when acquiring the number of target particles contained in the measurement sample, the target particles contained in the measurement sample can be appropriately evaluated based on the success or failure of the measurement. For example, if the target particles are rare cells, and the disease state is determined based on the number of target particles, the disease state can be properly determined based on the number of target particles if the measurement is performed properly. In this way, for example, in the determination of a disease state, it is possible to reduce false negatives caused by a measurement failure due to the small number of target particles.

Although a detection failure may occur each time the detection is performed, in the first embodiment, the success or failure of the measurement is determined based on the detection result of the actually performed measurement. Therefore, it can be determined whether the measurement has been correctly performed for each measurement of the measurement sample.

In the measurement success/failure determination step S25, whether the measurement by the sample measurement device 10 has been properly performed is determined using the detection rate based on the count value of the leukocytes occupying the majority of the measurement sample. As described above, in the first embodiment, the determination of the success or failure of the measurement of the target particles is performed by using another particle that is not originally an analysis target.

Note that the detection rate calculated by calculating the detection rate as shown in FIG. 10 may be a value corresponding to the estimated value, and the ratio is not limited to the ratio of the number of particles actually detected in the measurement of the measurement sample relative to the estimated value, as described above; for example, the ratio of the estimated value to the number of particles actually detected in the measurement of the measurement sample also may be used. In this case, the smaller the detection rate, the more appropriate the measurement can be determined. Therefore, in this case, if the detection rate is equal to or less than the threshold value Rth, the control unit 11 determines that the detection performed in the detection step S21 shown in FIG. 7 is appropriate and determines that the measurement is appropriate, whereas if the detection rate is larger than the threshold value Rth, it is determined that the detection performed in the detection step S21 shown in FIG. 7 is inappropriate and that the measurement is inappropriate. The detection rate is not limited to the ratio, and may be a value corresponding to a difference between the estimated value and the number of particles actually detected in the measurement of the measurement sample.

Returning to FIG. 7, in the analysis step S26, the control unit 11 analyzes the CTC as the target particles based on the detection result acquired in the detection step S21, that is, based on the fluorescence image stored in the storage unit 12.

Specifically, the control unit 11 extracts a bright point from a fluorescence image generated from a fluorescent stain that labels the Her2 gene and a fluorescence image generated from a fluorescent stain that labels the CEP17 gene. The control unit 11 divides, for each cell, the number of bright spots based on the Her2 gene by the number of bright spots based on CEP17, and when the calculated value is greater than 1, determines that the Her2 gene is amplified in the cell. Then, the control unit 11 determines a positive cell in which the Her2 gene has been amplified as CTC. Then, the control unit 11 acquires the number of determined CTCs, the ratio of CTCs to all cells imaged by the imaging unit 170, and the like.

Note that when the target particle is a vascular endothelial cell (CEC), the localization of NFκB, a protein contained in the CEC, in the cell is analyzed in the analysis step S26. Specifically, in the pretreatment, CEC is fluorescently labeled via a CD146-labeled antibody that specifically binds to an antibody expressed on CEC, and NFκB is fluorescently labeled via a labeled antibody that specifically binds to NFκB. In the detection step S21, fluorescence based on CEC is captured, and fluorescence based on NFκB is captured. Then, in the analysis step S26, CEC is detected based on the fluorescence image, and whether NFκB as a signal molecule localized in the nucleus is determined, and it is determined whether CEC is activated. When the target particle is CTC derived from lung cancer and the target site is cytokeratin, in the analysis step S26, CTC is detected based on the amount of cytokeratin present in the cytoplasm, and the number of CTC and the percentage of CTC are determined.

In the display step S27, the control unit 11 displays on the display unit 13 the image acquired in the detection step S21, the determination result acquired in the measurement success/failure determination step S25, the analysis result acquired in the analysis step S26, and the like.

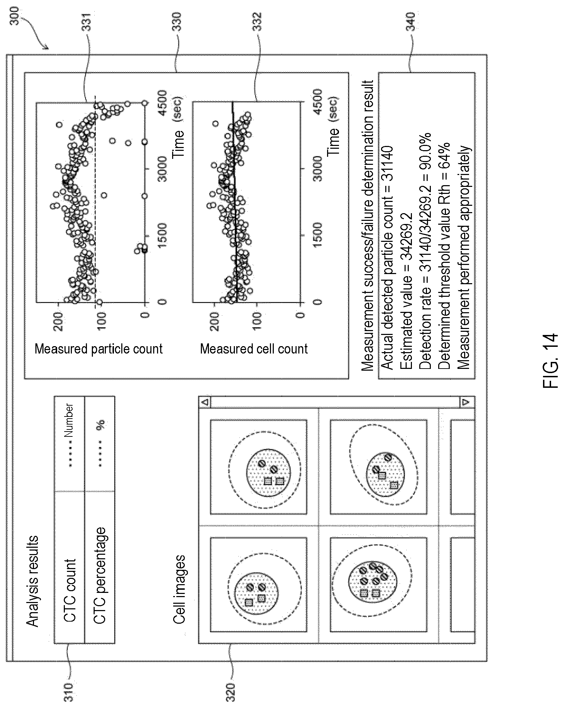

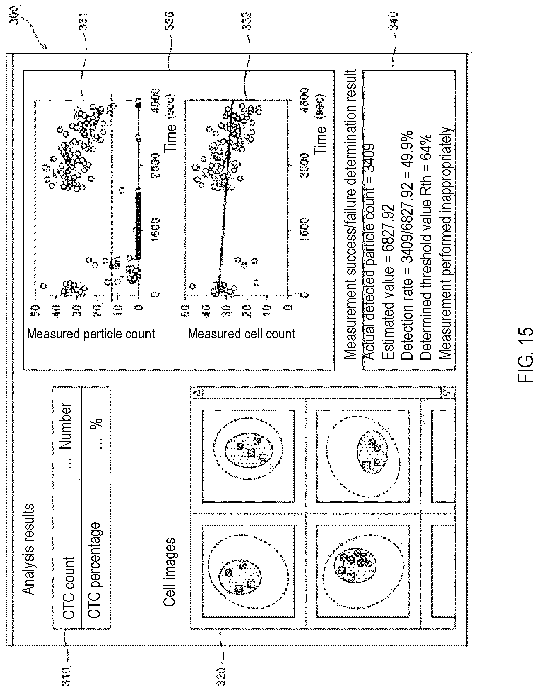

FIGS. 14 and 15 are schematic diagrams showing the configuration of the screen 300 displayed on the display unit 13 in the display step S27.

As shown in FIGS. 14 and 15, the screen 300 includes an analysis result region 310, a cell image region 320, a graph region 330, and a determination result region 340. FIG. 14 is an example of a screen 300 displayed for the measurement sample shown in the graphs of FIGS. 11A and 11B. FIG. 15 is an example of a screen 300 displayed for the measurement sample shown in the graphs of FIGS. 12A and 12B.

In the examples shown in FIGS. 14 and 15, the determination results of the success or failure of the measurement of the target particles are different. Hereinafter, the configuration of the screen 300 will be described with reference to FIGS. 14 and 15.

The analysis result area 310 displays values related to the number of CTCs, such as the number of CTCs and the ratio of CTCs acquired in the analysis step S26. The cell image area 320 displays the fluorescence image acquired in the detection step S21 shown in FIG. 7. A physician or the like can diagnose the patient from which the sample has been collected by referring to the value related to the number of CTCs displayed in the analysis result area 310 and the image of the cells displayed in the cell image area 320.

The graph area 330 displays graphs 331 and 332 of the number of measured particles. Graphs 331 and 332 are graphs showing the change over time in the number of detected particles. The graph 331 is a graph of the number of measured particles per unit time. In graph 331, a threshold value Nth for excluding particles in the generation of the approximation expression is indicated by a broken line. The graph 332 is a graph in which only the number of measured particles equal to or larger than the threshold Nth remain from the graph 331. The graph 332 shows an approximation expression generated based on the number of measured particles on the graph 332. As described above, when the graphs 331 and 332 are displayed on the display unit 13, the operator can grasp the detection result with reference to the graphs 331 and 332.

The determination result area 340 displays the number of particles actually detected, the estimated value, the detection rate, the threshold value Rth used in the determination, and the determination result of the success or failure of the measurement of the target particles. In the example shown in FIG. 14, the control unit 11 determines that the measurement of the target particles is appropriate, and displays the message "Measurement was correctly performed." in the region 340 since the detection rate is equal to or greater than the threshold value Rth. In this case, the operator can grasp that the reliability of the measurement result is high. On the other hand, in the example shown in FIG. 15, the control unit 11 determines that the measurement of the target particles was inappropriate, and displays the message "The measurement was not performed correctly. " in the determination result region 340 since the detection rate is less than the threshold value Rth. In this case, the operator can grasp that the reliability of the measurement result is low. As described above, when the measurement success/failure determination of the target particles is displayed on the display unit 13, the operator can smoothly grasp the success or failure of the measurement of the target particles with reference to the determination results.

A physician or the like can make a diagnosis based on the analysis result region 310 and the cell image region 320 when the measurement of the target particle is correctly performed by referring to the determination result of the success or failure of the measurement, whereas when the measurement is not performed correctly, the diagnosis based on the analysis result region 310 and the cell image region 320 can be deferred.

Note that when it is determined that the measurement of the target particles is inappropriate, the analysis result and the cell image are not displayed in the analysis result region 310 and the cell image region 320 on the screen 300 displayed on the display unit 13 in the display step S27. This can prevent a doctor or the like from diagnosing the patient based on an inappropriate result when the measurement of the target particles is inappropriate.

The measurement sample of the first embodiment contains, as other particles, leukocytes in which the number is larger than that of CTC, in addition to CTC as target particles. In the first embodiment described above, it is possible to determine the success or failure of the above-described measurement since the measurement sample includes a certain number of other particles. Therefore, for example, when the measurement sample contains only a few particles as a whole, such as when the measurement sample contains only rare cells CTC, it is preferable to increase the number of other particles in the measurement sample to some extent by mixing other particles such as beads or the like with the measurement sample. It is possible to determine the success or failure of the measurement as described above since the number of particles detectable in the detection step S21 shown in FIG. 7 increases when the number of particles in the measurement sample increases.

First Verification of Measurement Success/Failure Determination Method

Next, a first verification of the measurement success/failure determination method performed by the inventors will be described. In the first verification, whether the degree of appropriateness of the success or failure of the measurement was verified by comparing data acquisition rates which are the true detection rates using the detection rate acquired based on the procedure of the above-described first embodiment. The first verification includes the following procedures 1-1, 1-2, 1-3, 1-4, and 1-5.

1-1. Preparation of Measurement Sample

CTC obtained from the CTC model cell line was stained with a predetermined reagent. Then, CTC was added to PBS (phosphate buffered saline) so that 18,000 CTCs were contained therein to prepare a measurement sample. CTC counting was performed microscopically using a hemocytometer. Eight types of such measurement samples were prepared. Note that the measurement sample in this case does not include blood cells other than CTC, such as leukocytes.

1-2. Particle Count Measurement

The eight types of measurement samples prepared in Procedure 1-1 were respectively supplied to the sample measurement device 10 (see FIG. 2), and the measurement samples were measured by the sample measurement device 10. Here, the entire amount of the measurement sample was measured, and the measurement time was 4,500 seconds. The number of particles measured every 20 seconds was obtained based on the bright field image obtained by the measurement. Here, when measuring some of the eight kinds of measurement samples, the focus was shifted relative to the measurement sample flowing through the flow cell 110, thereby intentionally causing a failure in detection.

1-3. Calculation of Data Acquisition Rate

The data acquisition rate was calculated by dividing the total number of measured particles obtained by the measurement in Procedure 1-2 for each of the eight types of measurement samples by the original 18,000 CTCs. As described above, 18000 of the number of particles is a value obtained by actually counting the particles in the measurement sample, and is the number of particles actually supplied to the sample measurement device 10. As described above, when the true number of particles supplied to the sample measurement device 10 is known in advance, the calculated data acquisition rate becomes a value that truly reflects the detection rate of the particles detected by the sample measurement device 10. Therefore, the data acquisition rate can be said to be a true detection rate that can reliably determine the success or failure of the measurement.

1-4. Calculation of Detection Rate

The detection rate was calculated for each of the eight types of measurement samples in the same manner as in the procedure of Embodiment 1 described above.

Specifically, as described above, the average V1 of the number of measured particles having a number of measured particles of 1 or more was calculated, and the value V2 of 1SD of the number of measured particles having a number of measured particles of 1 or more was calculated. The average V1-value V2 was set as the threshold value Nth, and a graph of the measured particle number was generated while leaving only the measured particle number equal to or larger than the threshold value Nth. Then, an approximation expression was obtained based on the number of measured particles in the generated graph. Then, in the same manner as in the procedure of the first embodiment, the approximation expression was integrated from 0 seconds to 4500 seconds to calculate an estimated value. Then, the detection rate was obtained by dividing the total number of measured particles obtained by the measurement in the procedure 1-2 by the estimated value.

1-5. Creating a Scattergram

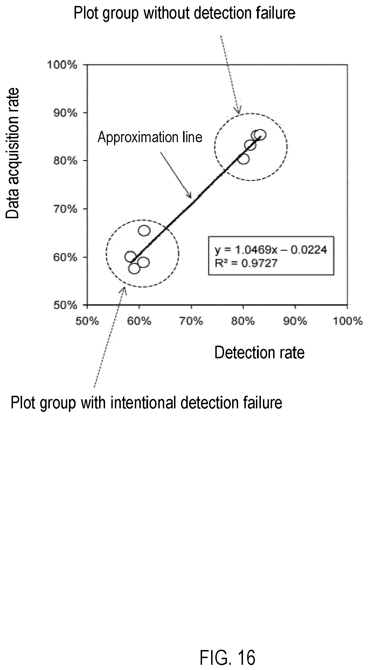

FIG. 16 is a scattergram of the data acquisition rate acquired in step 1-3 and the detection rate acquired in step 1-4. In the scattergram, points consisting of a data acquisition rate and a detection rate are plotted for eight types of measurement samples. In the scattergram of FIG. 16, as described above, when a number of measurement samples were measured, a detection failure was intentionally caused, so that the data acquisition rates of some measurement samples were low. A linear regression equation and a coefficient of determination were calculated based on the created scattergram. The linear regression equation was $y = 1.0469x-0.0224$, and the coefficient of determination was 0.727.

In the scattergram created through the above procedure, the points plotted for the eight types of measurement samples are generally arranged along a linear regression equation since the coefficient of determination is a value close to 1, and it can be said that there is a high correlation between the data acquisition rate and the detection rate.

Here, the estimated value used for calculating the detection rate in the procedure 1-4 is a value based on an approximation expression, and is the number of particles estimated to be supplied to the sample measuring apparatus 10. Originally, the measurement sample was visually inspected before measurement to obtain the true number of particles contained in the measurement sample in order to reliably obtain how many particles were detected in the sample measurement apparatus 10. However, it is shown in FIG. 16 that the detection rate based on the estimated value has a correlation with the data acquisition rate that reliably reflects the success or failure of the measurement. This indicates that the success or failure of the measurement can be properly determined even if the detection rate is used instead of the data acquisition rate when determining the success or failure of the measurement.

As shown in FIG. 16, it was found that the accuracy of measurement can be evaluated based on the detection rate since there is a correlation between the data acquisition rate and the detection rate. For example, in the case of the example shown in FIG. 16, it can be seen that the detection rate is preferably at least equal to or greater than the desired data acquisition rate in order to achieve the desired data acquisition rate. Therefore, according to the measurement success/failure determination step S25 shown in FIG. 7, it has been shown that the measurement success or failure can be determined based on whether the detection rate is greater than a predetermined threshold Rth.

Second Verification of Measurement Success/Failure Determination Method

Next, a second verification of the measurement success/failure determination method performed by the inventors will be described. In the second verification, it was verified whether the determination of the success or failure of the measurement based on the detection rate matches the success or failure of the measurement determined by visually checking the graph of the number of measured particles. In the second verification, it was verified that it was possible to determine whether CTC was properly detected by determining the success or failure of the measurement based on the detection rate. The second verification includes the following procedures 2-1, 2-2, 2-3, 2-4, and 2-5.

2-1. Preparation of Measurement Sample

CTC obtained from the CTC model cell line was stained with a predetermined reagent. Then, after counting the number of CTCs, the CTCs were added to the blood of healthy people to prepare measurement samples. CTC counting was performed using a microscope. Fifty-four kinds of such measurement samples were prepared. Note that the measurement samples in this case include non-CTC blood cells such as leukocytes.

2-2. Particle Count

Each of the 54 types of measurement samples prepared in step 2-1 was supplied to the sample measurement device 10 (see FIG. 2), and the measurement samples were measured by the sample measurement device 10. Here, the entire amount of the measurement sample was measured, and the measurement time was 4,500 seconds. The number of particles measured every 20 seconds was obtained based on the bright field image obtained by the measurement. FIGS. 11A and 11B and FIGS. 12A and 12B show the results of two measurement samples representing 54 types of measurement samples.

2-3. Calculation of Detection Rate

For each of the 54 types of measurement samples, the detection rate was calculated in the same manner as in the procedure of the above-described first embodiment and the procedure 1-4 of the first verification.

2-4. Detection Rate Distribution Chart Creation

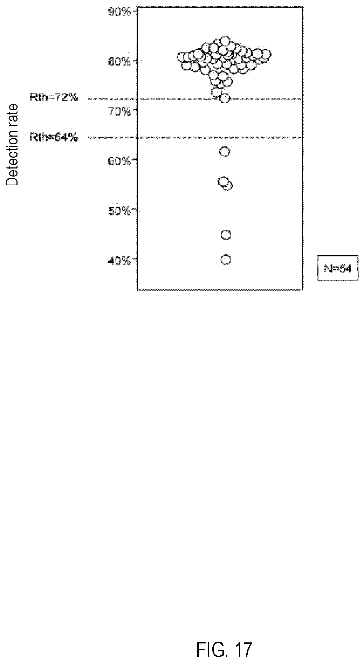

FIG. 17 is a distribution diagram created in the verification of the measurement success/failure determination method. As shown in FIG. 17, a distribution chart was created by plotting the detection rates obtained based on 54 types of measurement samples.
Here, as described above, the threshold value Rth to be compared with the detection rate is determined based on how many particles must be detected. For example, when 80% is set as the target value of the detection rate, the threshold value Rth may be set to 64%, assuming that the detection rate is preferably equal to or more than -2SD of 80%, that is, 64% or more. Alternatively, the threshold value Rth may be set to 72%, assuming that the detection rate is preferably -1 SD or more of 80%, that is, 72% or more. The setting of the threshold value Rth differs depending on the needs of the hospital, the operation of the testing facility, and the like.
In the distribution diagram of FIG. 17, when the threshold value Rth is set to either 64% or 72%, it was determined that the measurement was appropriate for 49 types of measurement samples, and the measurement was inappropriate for 5 types of measurement samples. The inventors confirmed the graph of the number of measured particles for the measurement sample determined to be appropriate for measurement, and found that the time period in which the number of measured particles was extremely small was short, as in FIGS. 11A and 11B. The inventors also confirmed a graph of the number of measured particles for the measurement sample determined to be inappropriate for measurement, and found that the time period when the number of measured particles was extremely small turned out to be long, as in FIGS. 12A and 12B.
The above findings suggest that the measurement success/failure determination method can appropriately determine the success or failure of the measurement.

2-5. Calculation of CTC Recovery Rate

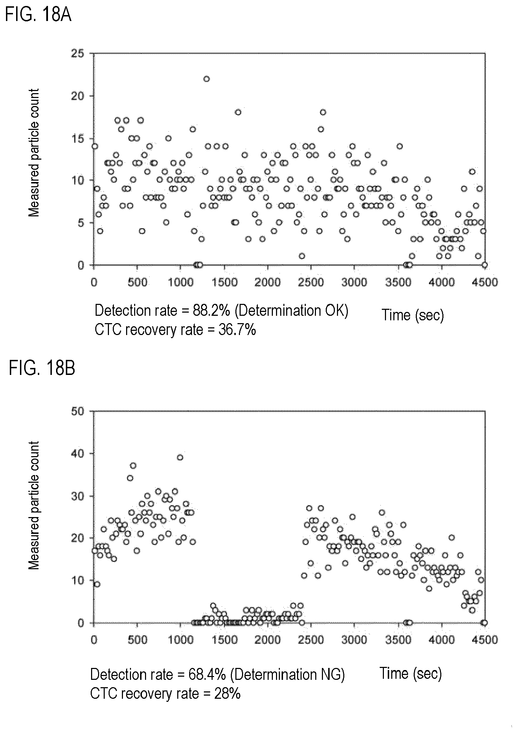

FIGS. 18A and 18B are graphs of the number of measured particles obtained for different measurement samples than the two measurement samples shown in FIGS. 11A and 11B and FIGS. 12A and 12B among 54 types of measurement samples. Here, when the threshold value Rth is set to 72%, in the case of FIG. 18A, the detection rate is 88.2%, so that it is determined that the detection is properly performed, whereas in the case of FIG. 18B it was determined that the detection was not properly performed since the detection rate was 68.4%.
Here, the number of CTCs was obtained for each of the measurement samples in FIGS. 18A and 18B in the same manner as in the analysis step S26 shown in FIG. 7. The CTC recovery rate was calculated by dividing the number of CTCs obtained in procedure 2-5 by the number of CTCs counted in step 2-1 for each of the measurement samples in FIGS. 18A and 18B. In the case of FIG. 18A, the CTC recovery rate was 36.7%, and in the case of FIG. 18B, the CTC recovery rate was 28%.
When the measurement is determined to be appropriate as shown in FIG. 18A, the CTC recovery rate is high, and when the measurement is determined to be inappropriate as shown in FIG. 18B, the CTC recovery rate is low. Therefore, it was suggested that when the determination of the success or failure of the measurement was performed as described above, there was a correlation between the success or failure of the measurement and the CTC recovery rate.

Second Embodiment

In the first embodiment, in the estimated value acquisition step S23 (see FIG. 7), the estimated value is generated based on the number of particles measured based on the image captured by the imaging unit 170 (see FIG. 5). On the other hand, in the second embodiment, in the estimated value obtained step S23, the estimated value is calculated based on the values of the detection signals based on the light received by the four photodetectors 152 as shown in FIG. 5.

In the second embodiment, in the measurement step S22 (see FIG. 7), the control unit 11 (see FIG. 2) integrates, for each unit time, the detection signal of the photodetector that has received the light having the wavelength $\lambda 14$ corresponding to the bright-field light to obtain the value of the detection signal. In the estimated value acquiring step S23 (see FIG. 7), the control unit 11 determines an approximation expression that approximates the change over time in the value of the detection signal detected for each unit time, and obtains the sum of the values of the detection signals defined by the approximation expression as an estimated value. In the detection rate acquiring step S24 (see FIG. 7), the ratio of the sum of the values of the detection signals actually detected in the measurement relative to the estimated value is obtained as the detection rate. Other aspects of the second embodiment are the same as those of the first embodiment.

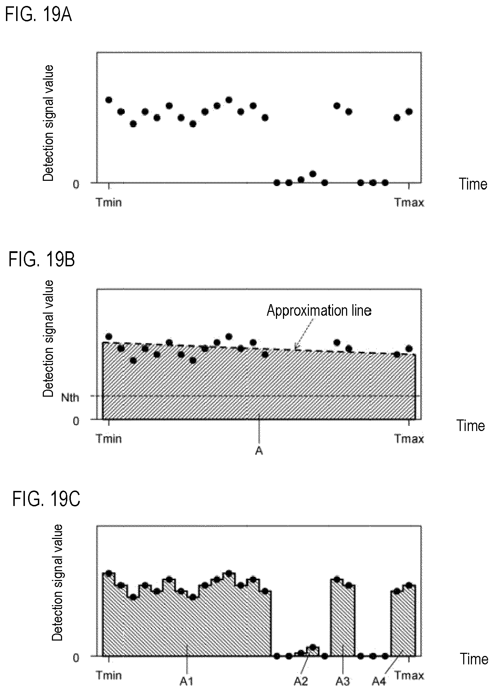

The acquisition of the estimated value and the detection rate according to the second embodiment will be described with reference to FIGS. 19A to 19C.

In the second embodiment, the detection signal based on the light of the wavelength $\lambda 14$ is integrated for every unit time, and the value of the detection signal is obtained for every unit time. In FIG. 19A, the horizontal axis indicates elapsed time, and the vertical axis indicates the value of the detection signal at each unit time. One plot on FIG. 19A is the detection signal of the photodetector integrated in the unit time, that is, the value of the detection signal in the unit time.

The control unit 11 acquires the estimated value and the detection rate based on the data corresponding to each plot in FIG. 19A in the same manner as in the first embodiment.

More specifically, the control unit 11 generates the threshold value Nth based on the data corresponding to FIG. 19A, and acquires an approximation expression based on the value of the detection signal equal to or larger than the threshold value Nth, as shown in FIG. 19B. Then, the control unit 11 calculates an estimated value by integrating the approximation expression from Tmin to Tmax. Note that, in this case as well, the control unit 11 calculates the estimated value by integrating the approximation expression reduced to 1/20 in the vertical axis direction from Tmin to Tmax since the value of the detection signal is obtained for each unit time. The estimated value calculated in this way means the estimated value of the sum of the expected detection signals in the current measurement, that is, the estimated value of the sum of the detected signals that would have been obtained if there was no detection failure. Note that this estimated value corresponds to the area A shown in FIG. 19B.

The control unit 11 also calculates a value (actually measured value) of the detection signal actually acquired in the measurement. The value of the detection signal actually acquired in the measurement is the sum of the values of the detection signal for each unit time. In the example shown in FIG. 19C, the sum of the values of the detection signals for each unit time corresponds to the area A1 + A2 + A3 + A4. Then, the control unit 11 calculates the detection rate based on the ratio of the expected sum of the detected signals (estimated value) to the sum of the detected signal values actually obtained in the measurement (actually measured values). For example, the control unit 11 obtains a detection rate by dividing the area corresponding to the actually measured value by the area corresponding to the estimated value. In the examples shown in FIGS. 19A to 19C, the detection rate corresponds to (A1 + A2 + A3 + A4) / A.

In the second embodiment, it also is possible to determine whether the measurement of the target particles is successful by comparing the detection rate with the threshold value Rth. In this way it possible to determine whether the number of detected particles is small due to the fact that the number of particles contained in the measurement sample is actually small, or whether the number of particles detected is small due to a detection failure.

Third Embodiment

Referring to FIG. 6, in the third embodiment, the first liquid sending unit 210 is configured to draw the measurement sample from the upper side of chamber 240 instead of drawing the measurement sample from the bottom of chamber 240 compared to the first embodiment. In the third embodiment, the number of particles in the measurement sample sent to the flow cell 110 decreases due to the sedimentation of the particles in the measurement sample, and the number of measurement particles may decrease over time. In this case, according to the measurement success/failure determination method of the first embodiment, there is a possibility that the measurement may be determined to be appropriate even though the measurement is inappropriate since the detection rate increases due to the lower estimated value.

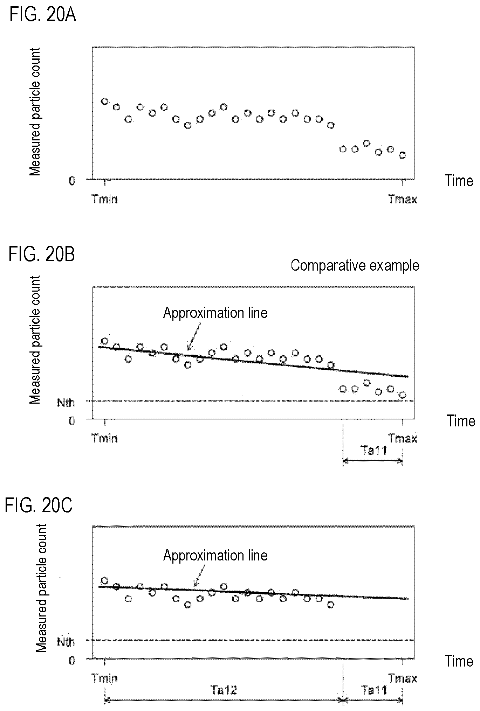

For example, an approximate expression as shown in FIG. 20B is generated if a graph of the number of measured particles as shown in FIG. 20A is obtained and an approximation expression is generated based on this graph. In this case, the area defined by the approximation expression, that is, the estimated value is reduced since the number of measured particles is reduced in the time zone Ta11. For this reason, the detection rate obtained by dividing the total number of measured particles in FIG. 20A by the estimated value increases, and the measurement is determined to be appropriate even though the measurement is inappropriate.

Therefore, in the third embodiment, an approximation expression is obtained by excluding a time zone after a predetermined timing at which it can be determined that the particle sedimentation is progressing in the entire time zone. The predetermined timing at which it can be determined that the particle sedimentation is progressing is determined based on a theoretical expression relating to the particle sedimentation, such as Stokes' formula. Note that other aspects of the third embodiment are the same as those of the first embodiment.

In the third embodiment as shown in FIG. 20C, the approximation expression is generated based on the number of measured particles in the time zone Ta12 before the predetermined timing, excluding the number of measured particles in the time zone Ta11 after the predetermined timing. In this way the estimated value of the area stipulated by the approximation expression of FIG. 20C is greater than that of FIG. 20B since the approximation expression of FIG. 20C which is nearer to parallel than the approximation expression generated in FIG. 20B. In the third embodiment, the detection rate is obtained by dividing the total number of measured particles in FIG. 20A by the estimated value obtained based on the approximation expression in FIG. 20C. The detection rate in this case is smaller than that in the comparative example of FIG. 20B. Therefore, according to the third embodiment, it is possible to determine a detection failure even when particle sedimentation occurs unintentionally by comparing the detection rate and the threshold value Rth as in the first embodiment.

Note that although the approximation expression is determined excluding the time zone after the predetermined timing in the entire time zone in the third embodiment, the present invention is not limited to this configuration inasmuch as the approximate expression may be obtained excluding a time zone before the predetermined timing when a failure can be expected to occur in the first half of the entire time zone. When a failure is expected to occur in a predetermined time zone among all time zones, an approximation expression may be obtained excluding the predetermined time zone.


Fourth Embodiment


In the graph of the number of measured particles in the fourth embodiment, the ratio of the time zone in which the particles are properly detected to the entire time zone is acquired as the detection rate. Other aspects of the fourth embodiment are the same as those of the first embodiment.

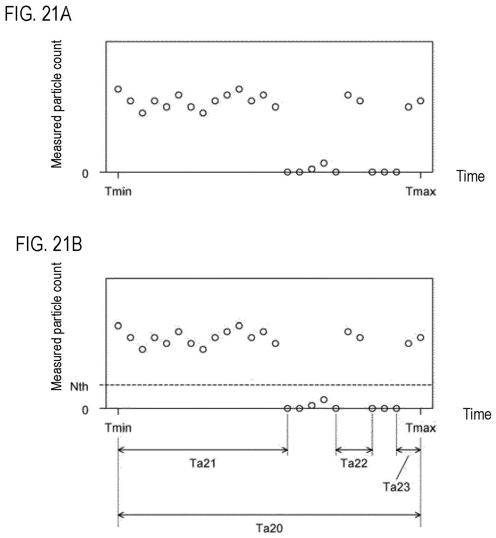

For example, when a graph of the number of measured particles as shown in FIG. 21A is obtained, the control unit 11 (refer to FIG. 2) performs the same operation as in the first embodiment, and a threshold value Nth for determining the possibility that a failure has occurred in detection is obtained, as shown in FIG. 21B. Then, the control unit 11 acquires the total time of the time period in which the number of measured particles is equal to or larger than the threshold value Nth. In the examples shown in FIGS. 21A and 21B, the total time of the time zone in which the number of measured particles is equal to or larger than the threshold value Nth is Ta21 + Ta22 + Ta23. Then, the control unit 11 obtains the detection rate by dividing the total time of the time zone in which the number of measured particles is equal to or greater than the threshold value Nth by the total time of all the time zones in the measurement of the measurement sample. In the examples shown in FIGS. 21A and 21B, the total time of all time zones is Ta20. Therefore, the detection rate in this case is (Ta21 + Ta22 + Ta23) / Ta20.

In the fourth embodiment as described above, success or failure of the measurement of the target particles also can be determined because the ratio of the time zone determined to be properly detected to all time zones is obtained as the detection rate.

Note that the control unit 11 also may acquire the detection rate by dividing the total time of the time zone in which the number of measured particles is less than the threshold value Nth by the total time of all the time zones. In the examples shown in FIGS. 21A and 21B, the detection rate in this case is {Ta20- (Ta21 + Ta22 + Ta23)}/Ta20. In this case, when the detection rate is equal to or less than the threshold value Rth, the measurement is determined to be appropriate, whereas when the detection rate is greater than the threshold value Rth, it is determined that the measurement is inappropriate. The detection rate may be (Ta21 + Ta22 + Ta23)/{Ta20- (Ta21 + Ta22 + Ta23)}. In the above detection rate calculation expression, the denominator and the numerator may be reversed.

In addition to the detection rate as described above, the success or failure of the measurement of the target particles also may be determined based on other statistical values obtained from the number of measured particles per unit time. For example, the difference in the number of measured particles between each time zone, the deviation of the number of measured particles in each time zone, and the standard deviation of the number of measured particles in the period including the entire time zone may be calculated, such that the success or failure of the measurement can be determined by comparing the calculated value with the threshold value.

When a measurement sample is prepared from whole blood, the number of particles contained in the measurement sample is unlikely to be extremely different for each measurement sample. When the number of particles contained in the measurement sample does not change significantly as described above, the success or failure of the measurement of the target particles may be determined based on the detection rate based on the time ratio as in the third embodiment. On the other hand, when the number of particles included in the measurement sample changes significantly, it is preferable to determine the success or failure of the measurement of the target particles and when it is desirable to more accurately determine the success or failure of the measurement of the target particles, the detection rate based on the ratio of the number of particles may be used as in the first embodiment to determine the success or failure of the measurement of the target particles.

**Claims**

1. A method for determining a success or failure of a measurement of a target particle contained in a measurement sample, the method comprising:

    detecting the target particle and other particle other than the target particle in the measurement sample; and
    determining the success or failure of the measurement of the target particle based on at least a detection result of the other particle.

2. The method according to claim 1, wherein
   determining the success or failure of the measurement comprises determining the success or failure of the measurement of the target particle based on the detection result of the target particle and the detection result of the other particle.

3. The method according to claim 1 or 2, wherein
   determining the success or failure of the measurement comprises determining the success or failure of the measurement of the target particle based on at least a detection result of the other particle per unit time.

4. The method according to any one of claims 1 to 3, further comprising

    calculating a value related to a detection rate of the target particle detected in the measurement based on at least the detection result of the other particle, wherein
    determining the success or failure of the measurement comprises determining the success or failure of the measurement of the target particle based on the value related to the detection rate.

5. The method according to claim 4, wherein
   calculating the value related to the detection rate comprises:

    calculating an estimated value related to the number of at least the other particle expected to be detected in the measurement based on the number of at least the other particle detected per unit time; and
    calculating the value related to the detection rate based on the estimated value and the number of at least the other particle actually detected in the measurement.

6. The method according to claim 5, wherein
   calculating the value related to the detection rate comprises calculating the value related to the detection rate based on a ratio between the estimated value and the number of at least the other particle actually detected in the measurement.

7. The method according to claim 5 or 6, wherein
   calculating the value related to the detection rate comprises calculating the value related to the detection rate based on a percentage of the number of at least the other particle actually detected in the measurement relative to the estimated value.

8. The method according to any one of claims 4 to 7, wherein
   determining the success or failure of the measurement comprises determining the success or failure of the measurement by comparing the value related to the detection rate with a predetermined threshold value.

9. The method according to claim 8, wherein the predetermined threshold value is changed according to a user input.

10. The method according to any one of claims 1 to 9, further comprising
    displaying a result of the determination of the success or failure of the measurement on a display unit.

11. The method according to claim 10, further comprising

displaying, on the display unit, a graph showing a change over time of the number of at least the other particle actually detected in the measurement.

12. The method according to any one of claims 4 to 9, wherein calculating the value related to the detection rate comprises:

calculating an estimated value related to the value of the detection signal expected to be detected in the measurement based on the value of the detection signal obtained from at least the other particle detected per unit time; and

calculating the value related to the detection rate based on the estimated value and the value of a detection signal actually detected in the measurement.

13. The method according to any one of claims 1 to 12, wherein detecting the target particle and the other particle comprises causing the measurement sample to flow through a flow path and detecting the target particle and the other particle in the measurement sample flowing through the flow path.

14. The method according to any one of claims 1 to 13, wherein

the target particle is a circulating tumor cell and the other particle is a leukocyte, and the method further comprises preparing the measurement sample by removing a part of leukocytes contained in a blood based on a difference in size of the blood circulating tumor cell and the leukocyte, wherein determining the success or failure of the measurement comprises determining the success or failure of the circulating tumor cell in the blood based on a detection result of leukocytes that has not been removed in the preparation of the measurement sample.

15. A sample measurement device that measures a target particle contained in a measurement sample, comprising:

a detection unit configured to detect the target particle and other particle other than the target particle in the measurement sample; and

a control unit configured to determine whether a measurement of the target particle is successful based on at least the detection result of the other particle.

Outline of Measurement Success/Failure Determination Method

```
        ┌──────────────┐
        │    START     │
        └──────┬───────┘
               │          S1
        ┌──────▼───────┐
        │              │
        │    Detect    │
        │              │
        └──────┬───────┘
               │          S2
        ┌──────▼───────┐
        │ Determine measurement │
        │  success or failure   │
        └──────┬───────┘
               │
        ┌──────▼───────┐
        │     END      │
        └──────────────┘
```

FIG. 1

Measurement sample

10

Sample measurement device

11 — Control unit

12 — Storage unit

Detection unit — 100

13 — Display unit

14 — Input unit

FIG. 2

Pretreatment sequence

```
        ┌─────────────────┐
        (     START       )
        └─────────────────┘
                 │          S11
                 ▼
    ┌────────────────────────────┐
    │       Collect sample       │
    └────────────────────────────┘
                 │          S12
                 ▼
    ┌────────────────────────────┐
    │    Erythrocyte hemolysis   │
    └────────────────────────────┘
                 │          S13
                 ▼
    ┌────────────────────────────┐
    │  First centrifugal separation │
    └────────────────────────────┘
                 │          S14
                 ▼
    ┌────────────────────────────┐
    │     Remove other particles │
    └────────────────────────────┘
                 │          S15
                 ▼
    ┌────────────────────────────┐
    │  CTC fluorescence labeling │
    └────────────────────────────┘
                 │          S16
                 ▼
    ┌────────────────────────────┐
    │ Second centrifugal separation │
    └────────────────────────────┘
                 │
                 ▼
        ┌─────────────────┐
        (      END        )
        └─────────────────┘
```

FIG. 3

FIG. 4A

FIG. 4B

A-A' Cross section view

FIG. 4C

1st sample
(to outlet 531)

2nd sample
(to outlet 532)

Pump

EP 3 699 574 A1

FIG. 5

EP 3 699 574 A1

FIG. 6

27

Operation of sample measurement device

```
        ┌─────────────┐
        (   START     )
        └──────┬──────┘
               │        S21
        ┌──────▼──────┐
        │   Detect    │
        └──────┬──────┘
               │        S22
        ┌──────▼──────┐
        │   Count     │
        └──────┬──────┘
               │        S23
        ┌──────▼──────┐
        │ Acquire estimated value │
        └──────┬──────┘
               │        S24
        ┌──────▼──────┐
        │ Acquire detection rate │
        └──────┬──────┘
               │        S25
        ┌──────▼──────┐
        │ Determine success/failure │
        │    of measurement │
        └──────┬──────┘
               │        S26
        ┌──────▼──────┐
        │ Analysis process │
        └──────┬──────┘
               │        S27
        ┌──────▼──────┐
        │ Display process │
        └──────┬──────┘
               │
        ┌──────▼──────┐
        (     END     )
        └─────────────┘
```

FIG. 7

FIG. 8A

Measured particle count

0

Tmin

Ta31    Ta32

Tmax    Time

FIG. 8B

Measured particle count

Approximation line

Nth

0

Tmin            A            Tmax    Time

FIG. 8C

Measured particle count

0

Tmin    A1            A2    A3    A4    Tmax    Time

Acquire estimated value

S101

Calculate average value V1

S102

Calculate 1SD value V2

S103

Calculate threshold value Nth

S104

Acquire approximation
expression

S105

Calculate estimated value

RETURN

FIG. 9

Acquire detection rate

S111

Calculate actual
measurement value

S112

Calculate detection rate

RETURN

FIG. 10

FIG. 11A : Example of determining measurement is appropriate

FIG. 11B: Example of determining measurement is appropriate

FIG. 12A: Example of determining measurement is inappropriate

FIG. 12B: Example of determining measurement is inappropriate

Determine measurement
success/failure

S121

Read out threshold value Rth

S122

Determination
(Detection rate equal to
or greater than threshold value
Rth?)

NO

YES

S123

Determine success
(determine measurement is
appropriate)

S124

Determine failure
(determine measurement is
inappropriate)

RETURN

FIG. 13

FIG. 14

EP 3 699 574 A1

Analysis results

| | |
|---|---|
| CTC count | ... Number |
| CTC percentage | ... % |

Cell images

Measured particle count

50
40
30
20
10
0

0    1500    3000    4500
Time (sec)

Measured cell count

50
40
30
20
10
0

0    1500    3000    4500
Time (sec)

Measurement success/failure determination result

Actual detected particle count = 3409
Estimated value = 6827.92
Detection rate = 3409/6827.92 = 49.9%
Determined threshold value Rth = 64%
Measurement performed inappropriately

300
310
320
330
331
332
340

FIG. 15

FIG. 16

FIG. 17

FIG. 18A

Detection rate = 88.2% (Determination OK)     Time (sec)
CTC recovery rate = 36.7%

FIG. 18B

Detection rate = 68.4% (Determination NG)     Time (sec)
CTC recovery rate = 28%

EP 3 699 574 A1

FIG. 19A

FIG. 19B

FIG. 19C

FIG. 20A

FIG. 20B

Comparative example

FIG. 20C

FIG. 21A

FIG. 21B

FIG. 22

Europäisches
Patentamt

European
Patent Office

Office européen
des brevets

# EUROPEAN SEARCH REPORT

Application Number

EP 20 15 7372

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | EP 3 002 581 A1 (SYSMEX CORP [JP])<br>6 April 2016 (2016-04-06)<br>* paragraph [0001]; figures 1,2,4-8 *<br>* paragraph [0006] - paragraph [0007] *<br>* paragraph [0010] - paragraph [0028] *<br>* paragraph [0037] - paragraph [0063] *<br>----- | 1-3,10,<br>11,13,15<br>4-9,12,<br>14 | INV.<br>G01N15/14<br>G01N35/00<br>G01N15/10<br>G01N1/40 |
| A | EP 3 096 125 A1 (SYSMEX CORP [JP])<br>23 November 2016 (2016-11-23)<br>* paragraph [0002] - paragraph [0005];<br>claim 1; figure 1 *<br>* paragraph [0008] - paragraph [0034] *<br>----- | 1-15 | |
| A | KR 2018 0058052 A (SYSMEX CORP [JP])<br>31 May 2018 (2018-05-31)<br>* paragraph [0001] - paragraph [0002];<br>figures 1,12,13 *<br>* paragraph [0008] - paragraph [0036] *<br>* paragraph [0081] - paragraph [0090] *<br>----- | 1-15 | |
| A | EP 3 299 812 A1 (UNIV WASHINGTON [US])<br>28 March 2018 (2018-03-28)<br>* paragraph [0003] - paragraph [0016] *<br>* paragraph [0060] - paragraph [0075] *<br>----- | 1-15 | TECHNICAL FIELDS<br>SEARCHED (IPC)<br><br>G01N |

The present search report has been drawn up for all claims

| Place of search | Date of completion of the search | Examiner |
|---|---|---|
| The Hague | 3 July 2020 | Foster, Keir |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

&: member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 20 15 7372

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2020

| Patent document cited in search report | | | Publication date | Patent family member(s) | | | Publication date |
|---|---|---|---|---|---|---|---|
| EP | 3002581 | A1 | 06-04-2016 | CN | 105466840 | A | 06-04-2016 |
| | | | | EP | 3002581 | A1 | 06-04-2016 |
| | | | | JP | 6342285 | B2 | 13-06-2018 |
| | | | | JP | 2016070833 | A | 09-05-2016 |
| | | | | US | 2016091428 | A1 | 31-03-2016 |
| EP | 3096125 | A1 | 23-11-2016 | CN | 106168571 | A | 30-11-2016 |
| | | | | EP | 3096125 | A1 | 23-11-2016 |
| | | | | JP | 6713730 | B2 | 24-06-2020 |
| | | | | JP | 2016217888 | A | 22-12-2016 |
| | | | | KR | 20160137359 | A | 30-11-2016 |
| | | | | US | 2016340636 | A1 | 24-11-2016 |
| | | | | US | 2020032196 | A1 | 30-01-2020 |
| KR | 20180058052 | A | 31-05-2018 | NONE | | | |
| EP | 3299812 | A1 | 28-03-2018 | AU | 2010236572 | A1 | 10-11-2011 |
| | | | | AU | 2015234379 | A1 | 29-10-2015 |
| | | | | CA | 2758382 | A1 | 21-10-2010 |
| | | | | CN | 102460154 | A | 16-05-2012 |
| | | | | CN | 105242004 | A | 13-01-2016 |
| | | | | EP | 2419726 | A2 | 22-02-2012 |
| | | | | EP | 2990795 | A1 | 02-03-2016 |
| | | | | EP | 3299812 | A1 | 28-03-2018 |
| | | | | ES | 2638861 | T3 | 24-10-2017 |
| | | | | JP | 5734274 | B2 | 17-06-2015 |
| | | | | JP | 6182168 | B2 | 16-08-2017 |
| | | | | JP | 6576374 | B2 | 18-09-2019 |
| | | | | JP | 2012523572 | A | 04-10-2012 |
| | | | | JP | 2015083992 | A | 30-04-2015 |
| | | | | JP | 2017096982 | A | 01-06-2017 |
| | | | | KR | 20120030362 | A | 28-03-2012 |
| | | | | US | 2012129190 | A1 | 24-05-2012 |
| | | | | US | 2019120857 | A1 | 25-04-2019 |
| | | | | WO | 2010120818 | A2 | 21-10-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017116558 A